(19) Europäisches Patentamt
European Patent Office
Office européen des brevets

(11) Publication number : **0 359 529 B1**

(12) # EUROPEAN PATENT SPECIFICATION

(45) Date of publication of patent specification : **03.03.93 Bulletin 93/09**

(51) Int. Cl.$^5$ : **A61K 37/02,** C07K 7/64

(21) Application number : **89309257.7**

(22) Date of filing : **12.09.89**

(54) **Method for the control of pneumocystis carinii.**

(30) Priority : **12.09.88 US 242767**
**16.09.88 US 246042**
**30.08.89 US 398889**

(43) Date of publication of application : **21.03.90 Bulletin 90/12**

(45) Publication of the grant of the patent : **03.03.93 Bulletin 93/09**

(84) Designated Contracting States : **AT BE CH DE ES FR GB IT LI LU NL SE**

(56) References cited :
**EP-A- 0 032 009**
**EP-A- 0 311 193**
**EP-A- 0 311 194**
**DE-A- 2 803 581**

(73) Proprietor : **MERCK & CO. INC.**
**126, East Lincoln Avenue P.O. Box 2000**
**Rahway New Jersey 07065-0900 (US)**

(72) Inventor : **Schmatz, Dennis M.**
**306 Elizabeth Avenue**
**Cranford New Jersey 07016 (US)**

(74) Representative : **Hesketh, Alan, Dr. et al**
**European Patent Department Merck & Co., Inc. Terlings Park Eastwick Road**
**Harlow Essex, CM20 2QR (GB)**

EP 0 359 529 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

Pneumocystis carinii is an opportunistic organism which is widely prevalent but generally dormant until bodily defenses of the host are compromised whereupon it propagates in the host causing disease. Generally it is present in the lungs although extrapulmonary infections have been reported. Lung infections with this organism in immunocompromised individuals generally lead to pneumonia and are usually fatal if untreated. It has been suggested that patient to patient transmission also may occur in immunocompromised individuals. The immunocompromised state of individuals is usually associated with genetic defects, lymphoproliferative diseases, or with conditions resulting from cancer therapy, or treatment with immunosuppressive drugs, or as a consequence of AIDS. Most AIDS patients eventually contract Pneumocystis carinii pneumonia (PCP) and have accounted for the majority of the recent cases of this disease. Left untreated, PCP is almost always fatal.

The current method of treatment for P. carinii pneumonia is trimethoprim/sulfamethoxazole or pentamidine. Treatment with trimethoprim/sulfamethoxazole (TMP/SMZ) is associated with a high level of toxic side effects including rash, elevated liver function, nausea and vomiting, anenia, creatine elevation, and in extreme cases Stevens-Johnson syndrome. Side effects from TMP/SMZ are much more prevalent in patients with AIDS. Treatment with pentamidine is also associated with a high level of toxic side effects including renal failure, hepatotoxicity, hypoglycemia, hematologic abnormalities and pain or abscess at the injection site. The mortality due to treatment can reach 20 to 30 percent. An improved method for the treatment of P. carinii pneumonia in immune-compromised patients is greatly needed.

DE-A-2803581 describes a family of derivatives of the cyclic peptide echinocandin B, which are stated to have antimycotic activity.

EP-A-0032009, meanwhile, describes a related class of semi-synthetic cyclic peptides, which are stated to have antifungal activity.

### DESCRIPTION OF THE INVENTION

The present invention is directed to the use of an anti-infection or infection controlling amount of a cyclohexapeptide compound for the preparation of a medicament, useful for treatment or prevention of Pneumocystis carinii infections in mammals.

The compounds within the scope of the present invention include a fungal metabolite as hereinafter detailed, known echinocandin type of antibiotics which are lipophilic cyclohexapeptides having a fatty acid side chain, simple derivatives of the echinocandin type antibiotics such as side chain dihydro- and tetrahydro- reduction products, ring ether and ester derivatives and semi-synthetic products in which the cyclohexapeptide ring is retained but in which the fatty acid side chain is replaced. Most of the compounds described in the literature are taught to be antifungal agents.

The compounds suitable for the practice of the present invention which include not only natural antibiotic compounds but also simple derivatives of natural antibiotic compounds and semi-synthetic compounds as are hereinafter fully detailed may be represented by the formula (I).

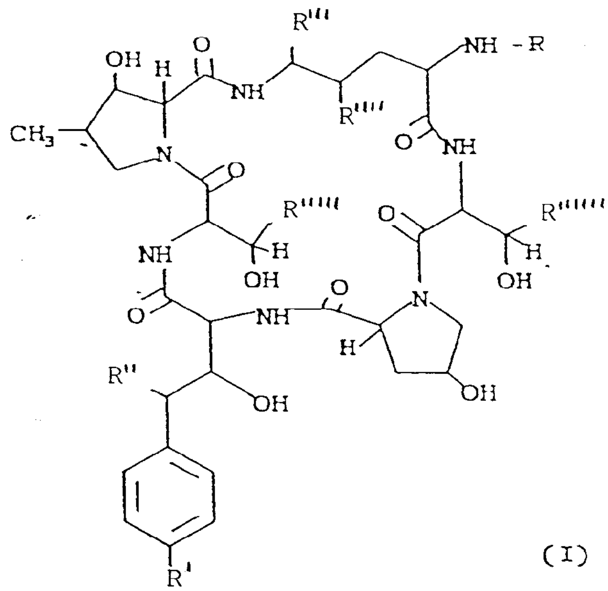

(I)

In this and succeeding formulas

R′ is H or OH;

R″ is H or OH;

R‴ is H, OH, -O-alkyl($C_1$-$C_6$), -O-benzyl, or

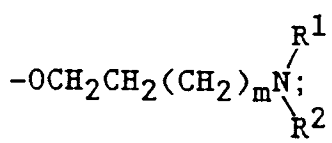

wherein $R^1$ is H, $C_1$-$C_{12}$ alkyl, $C_3$-$C_7$ cycloalkyl, hydroxyethyl, $C_{1-6}$ alkoxyethyl, furylmethyl, tetrahydrofurylmethyl, phenyl, phenylalkyl wherein the phenyl may be substituted with halo, hydroxy, $C_{1-6}$ alkyl and $C_{1-6}$ alkoxy; $R^2$ is hydrogen or $C_{1-6}$ alkyl; or $R^1$ and $R^2$ together form $-(CH_2)_2Q(CH_2)_2-$ wherein Q is $-CH_2-$,-NH- or -N-($C_{1-6}$)-alkyl; and m is from 0 to 4;

R⁗ is H or OH;

R′′′′′ is H, $CH_3$ or $CH_2CONH_2$; and

R′′′′′′ is H or $CH_3$

R is

(A)

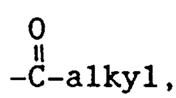

from 6 to 24 carbon atoms, or

(B)

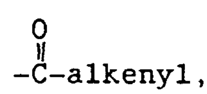

from 6 to 24 carbon atoms,

(C) when R′, R″, R‴ and R⁗ are OH, and R′′′′′ and R′′′′′′ are $CH_3$ then

(i)

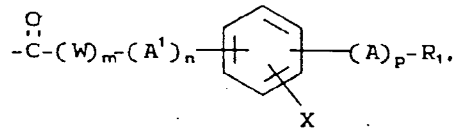

(ii)

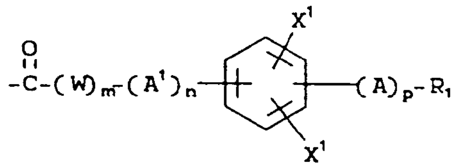

or
(iii)

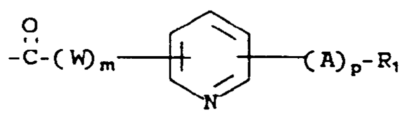

wherein

A is divalent oxygen, sulfur, sulfinyl or sulfonyl;

$A^1$ is divalent oxygen, sulfur, sulfinyl, sulfonyl or -NH-;

X is hydrogen, chloro, bromo, iodo, nitro, $C_1$-$C_3$ alkyl, hydroxy, $C_1$-$C_3$ alkoxy, mercapto, $C_1$,$C_3$ alkylthio, carbamyl or $C_1$-$C_3$ alkylcarbamyl;

$X^1$ is chloro, bromo or iodo;

$R_1$ is hydrogen, $C_1$-$C_{18}$alkyl or $C_2$-$C_{18}$alkenyl;

W is $C_1$-$C_{10}$alkylene or $C_2$-$C_{10}$alkenylene;

m, n and p are 0 or 1, but if m is 0, n must be 0;

provided that the sum of the carbon atoms in the $R_1$ and W groups must be greater than 4 but cannot exceed 21; that when X is mercapto A and $A^1$ cannot be sulfinyl or sulfonyl; and that when A and $A^1$ are sulfinyl or sulfonyl, they must be in equal oxidation states;

(D) when R′ is OH, R″ R‴ and R⁗ are H, then

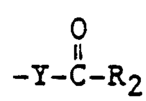

wherein Y is a divalent aminoacyl radical of the formula

a)

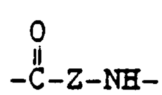

wherein Z is $C_1$-$C_{10}$alkylene or $C_5$-$C_6$cycloalkylene

b)

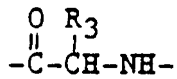

wherein $R_3$ is hydroxyethyl, hydroxyethyl, mercaptomethyl, mercaptoethyl, methylthioethyl, 2-thienyl, 3-indolomethyl, phenyl, benzyl, halophenyl, halobenzyl, $C_1$-$C_3$ alkylphenyl, $C_1$-$C_3$ alkylbenzyl, $C_1$-$C_3$ alkylthiophenyl, $C_1$-$C_3$ alkylthiobenzyl, carbamylphenyl, carbamylbenzyl, $C_1$-$C_3$ alkylcarbamylphenyl or $C_1$-$C_3$ alkylcarbamylbenzyl;

(c)

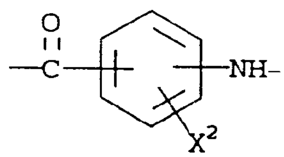

wherein $X^2$ is hydrogen or halo.

The term "alkyl" is meant a univalent saturated straight chain or branched chain hydrocarbon radical. The term "alkenyl" is meant an univalent, unsaturated, straight chain or branched chain hydrocarbon radical containing not more than three double bonds. The term "halo" is meant chloro, bromo, fluoro and iodo.

The compounds useful in the method of treatment for or prevention of Pneumocystis carinii infections in mammals according to the present invention are generally white crystalline or powdery substances. They are generally of good solubility in alcohols such as methanol and ethanol, or poor solubility in water and in hydrocarbons such as hexane and of intermediate solubility in many common solvents. The solubility properties as well as other properties of many of the naturally occurring cyclohexapeptides are summarized in the CRC Handbook of Antibiotic Compounds, Vol IV, Part I, pp 355-367, CRC Press, Inc. Boca Raton, Florida, 1980.

An especially preferred compound is a novel fungal metabolite in which R′, R″, R‴ and R″″ are OH, R‴‴ is $-CH_2CONH_2$, R″‴″ is $CH_3$ and R is

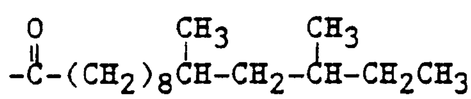

represented by formula IA

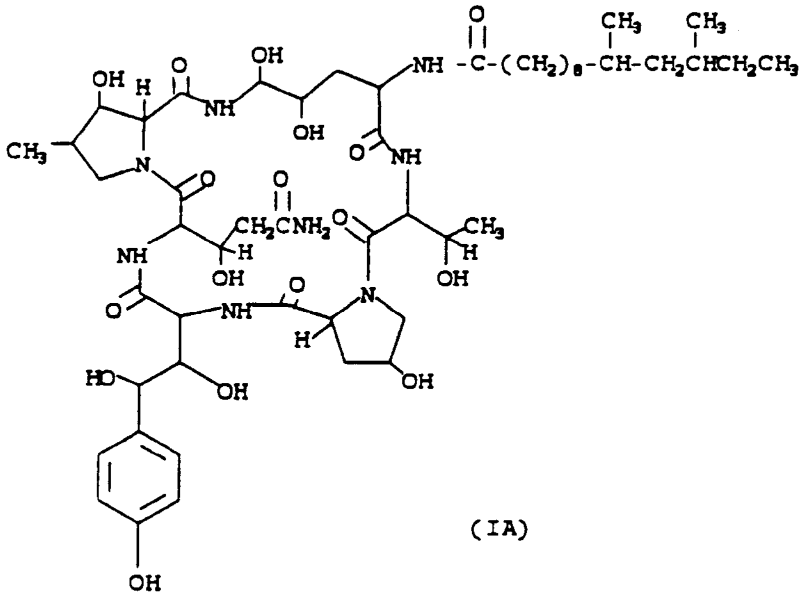

(IA)

The compound may be named 1-[4,5-dihydroxy-N2-(10,12-dimethyl-1-oxotetradecyl)-L-ornithine-5-(threo-3-hydroxy- 1-glutamine)echinocandin B but hereinafter, for convenience, will be referred to as Compound IA. Especially preferred is the isomer named 1-[(4R,5R)-4,5-dihydroxy-N2-(10,12-dimethyl-1-oxo-tetradecyl)-L-ornithine-5-(threo-3-hydroxy- 1-glutamine)echinocandin B. The preparation and properties of Compound IA are hereinafter described and are also the subject of copending applications Serial No. 105,795, filed October 7, 1987, corresponding to EP 311193, and Serial No. 105,797, filed October 7, 1987, corresponding to EP 311194, and are more fully described therein.

Compound IA, the preferred compound, is a white solid which may be characterized by the following physical properties:

a decomposition temperature of 206-214°C;

an empirical formula of $C_{51}H_{82}N_8O_{17}$ determined by high resolution FAB (Fast Atom Bombardment mass spectrometric measurement, calculated for $C_{51}H_{82}N_8O_{17}$ + Li = 1085.5958, found = 1085.6146);

an amino acid composition as determined by gas chromatogram/mass spectra of the trimethylsilyl derivative of the total acid hydrolysates of one equivalent each of threonine, hydroxyproline, methylhydroxyproline, and hydroxyglutamic acid.

$^1$H NMR Spectra in $CD_3OD$ at 400 MHz as seen in Figure 1; and

$^{13}$C NMR chemical shifts obtained in $CD_3OD$ at 100 MHz as follows: 11.20, 11.64, 19.75, 20.25, 20.78, 27.00, 28.07, 30.33, 30.37, 30.61, 30.76, 31.19, 31.29, 32.94, 34.83, 36.69, 38.10, 38.54, 39.07, 39.53, 45.93, 51.39, 53.01, 55.59, 56.31, 57.11, 58.35, 62.43, 68.18, 70.08, 70.55, 70.61, 71.26, 73.94, 75.72, 75.84, 76.86, 116.06(x2), 129.43(x2), 132.86, 158.22, 168.80, 172.16, 172.35, 172.40, 173.12, 174.24, 175.47, 176.88 ppm.

The compound is soluble in a variety of organic solvent such as methanol, ethanol, dimethylformamide, dimethyl sulfoxide, and the like.

The production of Compound IA, a novel compound, is described hereinafter in detail. The other compounds useful in the present invention may be prepared in a similar manner by substituting an appropriate microorganism or by other procedures as subsequently described.

Compound IA may be produced by cultivating a strain of microorganism designated MF 5171 in the culture collection of Merck & Co., Rahway, N.J. and identified as Zalerion arboricola and recovering said agent from the culture medium. A sample of the culture capable of producing the Compound IA has been deposited under the Budapest Treaty in the Culture Collection of the Americal Type Culture Collection at 12301 Parklawn Drive, Rockville, MD 20852. The sample has been assigned the accession number ATCC 20868.

The colonial and morphological description of ATCC 20868 are set forth below:

A. Morphological description

Globose, approximately 6.0 microns in diameter, thick-walled, dark coloured structures similar to chlamydospores develop along mycelium and often appear to be intercalary. These structures appear to divide, forming multi-celled groups of 4-8 cells which do not break up readily. On some media, strands of mycelia cluster together, forming rope-like structures and the multi-celled groups form large clusters as if held together by mucilaginous material.

B. Colonial description

1. Czapek-Dox agar

Colonies are slow growing, growth not extensive, flat with irregular edges. Mycelium is black, shiny; surface becomes dull, granular-appearing, black to greenish-brown as colony ages.

2. Corn agar

Colonies are slow-growing. Growth is not extensive. Mycelium is black, shiny surface becoming powdery, dull black as colony ages.

3. Potato-dextrose agar, Sabouraud maltose agar and yeast extract-malt extract-dextrose agar.

Colonies are slow growing. Growth is not extensive, is slightly raised in the center, radiately furrowed irregular edges except on Sabouraud-maltose agar where the edges are hyaline and regular. Mycelium is black, shiny; becomes dull powder, black as colony ages.

Although the invention is discussed hereinbelow principally with respect to the specific strain, it is well-known in the art that the properties of microorganisms may be varied naturally and artificially. Thus, all strains of the genus ATCC 20868 including varieties and mutants, whether obtained by natural selection, produced by the action of mutating agents such as ionizing radiation or ultraviolet irradiation, or by the action of chemical mutagens such as nitrosoguanidine, are contemplated to be within the scope of this invention.

Compound IA may be produced in a form adaptable for drug use by cultivating the strain ATCC 20868 of Zalerion arboricola in a nutrient medium until a substantial amount of antibiotic activity is detected in the culture medium, generally in the mycelium portion thereof, and thereafter recovering the active component from the fermentation mycelium in a suitable solvent, concentrating the solution of active component, and then subjected the concentrated material to chromatographic separation.

The fermentation is carried out in a medium containing sources of carbon and nitrogen assimilable by the microorganisms and generally low levels of inorganic salts. In addition, the medium may be supplemented with trace metals, although if complex sources of carbon and nitrogen are employed, they are usually present in the complex sources.

The sources of carbon include glycerol, sugars, starches and other carbohydrates or carbohydrate derivatives such as dextran, cerelose, as well as complex nutrients such as oat flour, corn meal, millet and corn. The exact quantity of the carbon source which is utilized in the medium will depend, in part, upon the other ingredients in the medium, but it is usually found that an amount of carbohydrate between 0.5 and 5 percent by weight of the medium is satisfactory. These carbon sources can be used individually or several such carbon sources may be combined in the same medium.

The sources of nitrogen include amino acids such as glycine, arginine, threonine, methionine and the like as well as complex sources such as yeast hydrolysates, yeast autolysates, yeast cells, tomato paste, soybean meal, casein hydrolysates, yeast extracts, corn steep liquors, distillers solubles, cottonseed meal, and meat extract. The various sources of nitrogen can be used alone or in combination in amounts ranging from 0.2 to 90 percent by weight of the medium.

Among the nutrient inorganic salts, which can be incorporated in the culture media are the customary salts capable of yielding sodium, potassium, magnesium, ammonium, calcium, phosphate, sulfate, chloride, or carbonate. Also included are trace metals such as cobalt, manganese, iron, molybdenum, zinc, and cadmium.

The media suitable for carrying out the fermentation may be solid or liquid.

Solid media may have a millet, corn, oats, soy bean or wheat base. One medium having a millet base is Medium 2 in Example A. Other representative solid media include the following:

| Media | Weight or Volume Per 250 ml Flask |
|---|---|
| Medium A | |
| Corn (cracked) | 10.0 g |
| Yeast extract | 0.5 g |
| Sodium tartrate | 0.1 g |
| Monosodium glutamate | 0.1 g |
| Corn oil | 0.1 ml |
| Ferrous sulfate•$7H_2O$ | 0.01 g |
| Water | 15-20 ml |
| Medium B | |
| Millet | 15 g |
| Yeast extract | 0.5 g |
| Sodium tartrate | 0.1 g |
| Ferric sulfate•$7H_2O$ | 0.01 g |
| Sucrose | 0.5 g |
| Alfalfa | 0.5 g |
| Corn oil | 0.1 ml |
| Water | 15 ml |
| Medium C | |
| Millet | 15 g |
| Yeast extract | 0.5 g |
| Sodium tartrate | 0.1 g |
| Ferric sulfate•$7H_2O$ | 0.01 g |
| Silica gel | 0.5 g |
| Alfalfa | 0.5 g |
| Monosodium glutamate | 0.1 g |
| Corn oil | 0.1 ml |
| Water | 15 ml |

| Medium D | |
|---|---|
| Millet | 15 g |
| Yeast extract | 0.5 g |
| Sodium tartrate | 0.1 g |
| Ferric sulfate•$7H_2O$ | 0.01 g |

In addition, media may be prepared by substituting wheat, barley, oats or soy bean for the millet or corn above.

Liquid media also may be employed. Fermentation on a larger scale is generally more conveniently carried out employing a liquid medium. It has been found that although conventional liquid media may be employed to obtain Compound IA, such media have not been found to be suitable for obtaining good yields of the desired antibiotic. However, by incorporating from about 6 to 9 percent by weight of glycerol, it has been found that good yields of the desired antibiotic compound may be obtained. Suitable liquid media include:

| Medium E | |
|---|---|
| Glycerol | 85 g |
| Pectin | 10 g |
| Peanut Meal | 4 g |
| Peptonized Milk | 4 g |
| Tomato Paste | 4 g |
| Corn Steep | 4 g |
| Lard Water | 4 g |
| Glycine | 2 g |
| $KH_2PO_4$ | 2 g |
| Distilled Water to | 1000 ml |

pH 7.0

| Medium F | |
|---|---|
| Dextrose | 10 g |
| Glycerol | 10 ml |
| Soy Flour | 4 g |
| Peptonized milk | 4 g |
| Tomato paste | 4 g |
| Lard water | 4 g |
| Potassium dihydrogen phosphate | 2 g |
| Cobalt chloride hexahydrate | 0.01 g |
| Distilled Water to | 1000 ml |

pH 7.0

For producing Compound IA, a fermentation medium containing ATCC 20868 is prepared by inoculating spores or mycelia of the antibiotic-producing organism into a suitable medium and then cultivating under aerobic conditions.

The procedure generally is first to inoculate a preserved source of culture from an agar slant containing nutrient medium into a nutrient seed-producing medium and to obtain, preferably through a two step procedure, growth of the organisms which serve as seeds in the production of the antipneumocystis agent.

In this process, a slant section of a preserved culture of ATCC 20868 is inoculated into an appropriate liquid nutrient seed medium of pH in the range 5 to 8.1, optimally 6 to 7.5, and the flasks incubated with or without agitation at temperatures in the range of from about 15°C to about 30°C, preferably 20° to 28°C. Agitation when employed, may be up to 400 rpm, preferably, about 200 to 220 rpm. The incubation is carried out over a period of from 2 to 30 days, preferably 2 to 14 days. When growth is abundant, usually between 2 and 5 days, the culture growth may be used to inoculate the production medium for the production of the antipneumocystis agent. Preferably however, a second stage fermentation is carried out, inoculating with a portion of the culture growth and then employing similar conditions but generally with shortened incubation period of about 1 to 3 days. The growth then is employed to inoculate the production medium.

The fermentation production medium inoculated with the culture growth is incubated for 3 to 30 days, usually 7 to 14 days with or without agitation. The fermentation may be conducted aerobically at temperatures ranging from about 20°C to about 40°C. For optimum results, it is most convenient to conduct these fermentations at a temperature in the range of from about 24°C to about 30°C. Temperatures of about 24°-28°C are most preferred. The pH of the nutrient medium suitable for producing the instant compounds can vary from about 5.0 to 8.5 with a preferred range of from about 6.0 to 7.5. After the appropriate period for the production of the desired compound or compounds, the latter is recovered from the fermentation medium as hereinafter more fully described.

The active material may be recovered from the fermentation medium by the steps comprising

(1) adding alcohol to said medium, stirring and filtering to recover the active component in the resulting aqueous alcoholic solution;

(2) concentrating the aqueous alcoholic solution to a small volume of primarily aqueous solution;

(3) intimately contacting the resulting concentrated alcoholic aqueous solution with a water-immiscible organic solvent to extract or partition the active component thereinto and concentrating, or subjecting the concentrated solution to HP-20 adsorption and elution; then

(4) subjecting the material recovered in Step (3) to at least one chromatographic separation, wherein in each chromatographic separation, the active component from the eluates exhibiting activity against Candida albicans are combined and concentrated to recover Compound IA.

The exact steps may vary somewhat depending on whether the fermentation had been carried out in liquid or solid medium, what solvent is employed and what adsorbent or combination of adsorbents is employed.

When the fermentation is carried out on solid medium, the first step may be carried out by adding an alcoholic solvent to the fermentation medium, thoroughly mixing, then filtering, recovering and concentrating the aqueous alcohol filtrate. Preferably, the concentrated filtrate is first back-extracted or washed with a lower aliphatic hydrocarbon solvent such as hexane or other alkane to remove alkane soluble impurities. The alkane washed filtrate may be extracted or partitioned with a water-immiscible oxygenated organic solvent and the resulting solution concentrated, then placed onto a column for at least one, generally several chromatographic separation steps. Suitable columns are silica gel, reverse phase and SEPHADEX LH-20.

When the fermentation is carried out in a liquid medium, in one method, the mycelial solids are filtered and recovered from the fermentation medium. Alcohol is added to the mycelial cake, and the mycelial solid thoroughly mixed with the alcohol, filtered, and the filtrate collected and concentrated. In an alternative method, the whole broth can be extracted by the addition of one volume of alkanol, preferably methanol, and filtered to remove solid impurities. The alkanol extract is then adsorbed on DIAION SP-207 or other commercially available styrene-divinylbenzene copolymer. A second dilution/HP-20 adsorption/elution step is utilized to concentrate the sample in preparation for chromatographic separations. Sometimes, a third dilution/HP-20 adsorption/elution step may be desirable for volume reduction.

The alcoholic solvent to be employed in the initial extraction of the active agent from the solid nutrient medium or from the mycelial pad may be any of the lower alcohols such as methanol, ethanol, isopropanol, and the like. Methanol is preferred.

The water-immiscible non-polar organic solvent useful for extracting or partitioning the active agent from the alkanol or methanol solution are esters, such as ethyl acetate, isopropyl acetate, butyl acetate, or ketones, such as methyl ethyl ketone. Lower aliphatic esters are preferred.

The chromatographic separation may be carried out by employing conventional column chromatography with non-ionic resin or by high performance liquid chromatography employing reverse phase resin. The fractions containing the antibiotic Compound IA may be detected by bioautography using Candida albicans. Generally, more than one chromatographic separation steps are employed. In a most preferred procedure, one or more separations are carried out employing column chromatography and a final separation is carried out employing high performance liquid chromatography (HPLC) with $C_{18}$ reverse phase resin.

When conventional column chromatography is employed for chromatographic separations, silica gel is the preferred adsorbent. Usually more than one chromatographic separation is required. Silica gel may be used in all the separations while employing different eluting agents. However, it may be combined advantageously with the use of a different adsorbent such as a dextran gel sold under the trade name of SEPHADEX LH-20 (Pharmacia). Other adsorbents such as alumina, styrene-divinylbenzene copolymers available commercially as DIAION HP-20, HP-30, HP-40, SP-207 (Mitsubishi Chemical Industries, Ltd.) and AMBERLITE XAD-2, XAD-4, XAD-16 (Rohm and Haas Co.) also may be employed.

In the fractionation and recovery of the active component by chromatography on silica gel, ester/alcohol mixtures with increasing concentration of alcohol provide good separations. A mixture of ethyl acetate and methanol has been found to be especially useful. These may be employed in isocratic, step gradient or continuous gradient systems. When a dextran adsorbent such as SEPHADEX LH-20, is employed, a chlorohydrocarbon/hydrocarbon/alcohol solvent system may be employed. A mixture of methylene chloride/hexane/methanol has been found to be especially useful.

In carrying out the HPLC separation, the alcohol solution containing material recovered from the conventional chromatography is concentrated and the residue dissolved in methanol and placed on a column packed with commercial reverse phase resin or on a column filled with silica gel/$C_{18}$ reverse phase resin prepared as amply reported in the literature. Alternatively, the alcohol solution may be diluted to 50 percent with water and pumped onto the column. The column is operated using methane/water (1:1 or optionally other ratios) at 800-2000 psi [$(5.5\text{-}13.8)\times10^6$ Pa] which produces a flow rate of about 20 ml/min. Separation is monitored at 210 nm.

The fractions are assayed for activity with Candida albicans. The product is recovered from the chromatographic procedures by combining the active fractions and concentrating under reduced pressure. The product then may be purified by conventional chromatography or preparative HPLC.

Other natural products which are embraced by the present invention include those which are described in the literature as echinocandins, aculeacins, mulundocandins, athlestain (also referred to as an echinocandin T), sporiofungin or by number designations. The structures in some cases are not completely identified but the compounds are known to be neutral cyclohexapeptides with fatty acid side chain.

The echinocandins include echinocandin-A (also known as A-32204-A) produced by Aspergillus nidulans-echinulatus (Swiss patent 568,386); echinocandin-B (also known as A-32204-B and SF-7810-F) produced by Asp. nidulans (Swiss 568,386) and Asp. rugulosus (Helv. Chim. Acta 62 11252 (1979); German 2,549,127; Belg. 834,289); echinocandin-C (also known as SL-7810-FII) produced by Asp. rugulosus (Ger. 2,549,127; Belg. 834,289); echinocandin-D (also known as SL-7810-FIII) produced by Asp. rugulosus (Ger. 2,549,127; Belg. 834,289).

Echinocandins B, C and D may be produced together on the fermentations of a strain of Aspergillus rugulosus. The major metabolite in the fermentation of Aspergillus rugulosus is echinocandin B, having a molecular formula of $C_{52}H_{81}N_7O_{16}$. Echinocandin C and D which are the minor metabolites differ in certain of the amino acids making up the cyclic peptide ring. Thus, echinocandin C, having a molecular formula of $C_{52}H_{81}N_7O_{15}$, contains 3-hydroxyhomotyrosine in place of the 3,4-dihydroxyhomotyrosine present in echinocandin B. Echinocandin D, having a molecular formula of $C_{52}H_{81}N_7O_{13}$, contains 3-hydroxyhomotyrosine and ornithine instead of 3,4-dihydroxyhomotyrosine and 4,5-dihydroxyornithine.

Echinocandin B also may be produced by the cultivation of Emericella nidulans as described in the working example.

The aculeacins include aculeacin-A, aculeacin-B, aculeacin-C, aculeacin-D, aculeacin-E, aculeacin-F, aculeacin-G, aculeacin-AA″, aculeacin-DA″, aculeacin-AG″, aculeacin-Aα and Aγ, and aculeacin-Dα and Dγ. The aculeacins may be produced by the fermentation of Aspergillus aculeatus (either liquid or solid culture) (preferably M4214 FERM-P 2324) thereafter extracting the mycelium with a water miscible organic solvent concentrating and purifying by chromatography as described in U.S. Patent 3,978,210 and 4,212,858 German Patent 2,509,820 and Belgian Patent 826,393.

Another cyclohexapeptide antibiotic found in the literature is mulundocandin (R′, R″, R‴, R″″ are OH, R‴‴ is H, R is $-CO(CH_2)_{10}-CH_3CH-CH_2CH_3$) produced by Asp. sydowic No. Y-30462 (J. Antibiotics 40, 275 and 40, 281 (1987)). The antibiotic, which is present both in the culture filtrate and mycelium may be isolated from the ethyl acetate extract of a culture filtrate by silica gel column chromatography and droplet counter current chromatography as more fully described by Roy et al, J. Antibiotics 40, 275-280 (1987).

Still another echinocandin type cyclopeptide reported in the literature is sporiofungin (R′, R″″ and R″″ are OH, R″ is H, R″″ is $CH_2CONH_2$, R‴‴ is H and R is a branched chain $C_{16}$ fatty acid radical,) which may be produced by cultivation of Cryptosporiopsis (WIPO publication, WO 82/00587) strain ATCC 20594 or NRRL 12192, on various conventional nutrient media as aerobic surface culture or immersion culture, and the mycelium separated from the culture broth and recovered in a conventional manner such as by homogenizing the mycelium with methanol, extracting from the methanol with a water-immersible organic solvent such as ethyl acetate, removing the solvent and purifying by chromatography as more fully described in WO 82/00587.

Other echinocandin type cyclohexapeptides include X-73 produced by Aspergillus rugulosus, (Ind. J.B. Biochem. 7, 81 (1970)), athlestain produced by Aspergillus niger, (Jap. J. Ant., 17 268 (1964), JP 66/12688; CA 65 19274; 70, 27607), S-31794-F-1 produced by Acrophialophora limonispora (U.S. 4,173, 629; Ger. 2,628,965), A-30912-A or A-22082 produced by Asp. nidulans and Asp. rugulosus (U.S. 4,074,246; 4,074,245; 4,288,549; Ger. 2,643,485; CA 87, 20612) A-30912-B, A-30912-C, and A-30912-D, produced by Asp. rugulosus (U.S. 4,024,245).

Compounds which are modifications of natural products include those in which the side chain, i.e., R, when an unsaturated fatty acid, had been modified by reduction. This may be carried out catalytically, by using Pd on carbon at atmospheric pressure.

Compounds in which R''' is -O-Alkyl ($C_1$-$C_6$) or -O-benzyl may be prepared by stirring together the corresponding compound in which R''' is OH with the appropriate alkanol under acidic conditions at ambient temperature for several hours or overnight to obtain the ether in the mixture and thereafter preferably quenching the reaction with dilute bicarbonate and recovering it by conventional procedures. In addition to the preparation in the working example, the preparation for a number of these compounds is more fully described in GB 2,050,385A.

Compounds in which R''' is

$$-O-CH_2-CH_2-(CH_2)_m-N\begin{matrix}R^1\\R^2\end{matrix}$$

wherein $R^1$ is hydrogen, alkyl or substituted alkyl as previously defined, $R^2$ is hydrogen or $C_1$-$C_4$ alkyl, or $R^1$ and $R^2$ together form $-(CH_2)_2-Q-(CH_2)_2-$ wherein Q is a connecting group such as $-CH_2-$, -NH- or -N(lower alkyl)- may be prepared by reacting a compound in which R''' is OH with a compound of the formula

$$HO-CH_2-CH_2-(CH_2)_m-N\begin{matrix}R^1\\R^2\end{matrix}$$

in the presence of an organic acid such as p-toluenesulfonic acid, methanesulfonic acid or mineral acid in an aprotic solvent such as dimethylformamide as more fully described in Belgian 851,310.

Semi-synthetic compounds in which the acyl side chain, i.e., R in above formula, has been modified from naturally occurring fatty acids to structurally distinctive acyl groups may be produced by first removing the fatty acid side chain and thereafter introducing a distinctive acyl group. The fatty acid side chain preferably is removed enzymatically. The enzyme which is useful for deacylation of the cyclohexapeptides to obtain a cyclopeptide nucleus is that produced by certain microorganisms of the family Actinoplanaceae, especially the microorganism Actinoplanes utahensis NRRL 12052 which is available from the Northern Regional Research Center, USDA, Agricultural Research Service, Peoria I11 61604, or as A. utahensis ATCC 14539 obtainable from the American Type Culture Collection, 12301 Parklawn Drive, Rockville, MD 20852.

The enzyme may be produced by growing Actinoplanaceae at temperatures between about 25°C and 30°C and a pH of between about 5.0 and 8.0, preferably between 6.0 and 7.0, with agitation and aeration for from about 40 to about 60 hours in a culture medium containing (a) an assimilable carbon source such as sucrose, glucose or glycerol, (b) a nitrogen source such as peptone, urea or ammonium sulfate (c) a phosphate source such as a soluble phosphate salt and (d) growth promoting inorganic salts.

In the deacylation, the cyclohexapeptide compound or substrate containing the cyclohexapeptide is added to the culture of Actinoplanaceae after the culture has been incubated for at least 48 hours. After addition of the substrate, the incubation of the culture is continued for about 24 hours or longer over temperatures in the range of from about 25°C to about 30°C.

The course of the reaction may be monitored by Candida albicans assay. The starting cyclohexapeptide compound is active against C. albicans but the deacylated nucleus compound is biologically inactive.

The deacylated nucleus compound then may be employed in the preparation of semi-synthetic compounds. Conventional acylation methods may be employed. In one preferred method, a 2,4,5-trichlorophenyl

ester of the desired acid is reacted with the deacylated nucleus compound in an inert solvent such as dimethylformamide at room temperature for about 15 to 18 hours. This may be illustrated with a specific example as follows:

A deacylated nucleus of a compound the following structure wherein in formula (IB), E is H (Compound IBi)

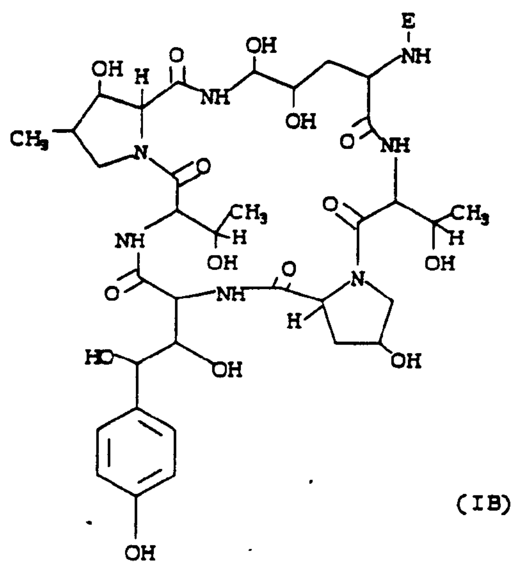

(IB)

may be prepared by first preparing a deacylating enzyme by the fermentation of Actinoplanes utahensis NRRL 12052 by first inoculating a slant with the organism, incubating the slant at 30°C for about 8 to 10 days and then using the slant growth to inoculate 50 milliliters of vegetative medium which is incubated at 30°C for about 72 hours with shaking. The incubated vegetative medium may be used to inoculate 400 millilitres of a second-stage vegetative medium having the same composition as the first stage vegetative medium and incubated at 30°C for about 48 hours with shaking. Thereafter, 800 millilitres of said medium may be used to inoculate 100 litres of production medium which is permitted to ferment at about 30°C for 42 hours with agitation and aeration to maintain dissolved oxygen level above 30 percent of air saturation at atmospheric pressure and to obtain therein the desired deacylating enzyme.

The medium for preparing the slant may be of the following composition: Baby oatmeal 60.0 g; yeast 2.5 g; $K_2HPO_4$ 1.0 g; Czepek's mineral stock 5.0 ml; agar 25.0 g; deionized water q.s. to 1 liter. Czepek's mineral stock is of the following composition: $FeSO_4 \bullet 7H_2O$ (in 2 ml. conc. HCl) 2 g; KCl 100 g: $MgSO_4 \bullet 7H_2O$ 100 g; deionized water to 1 liter with final adjusted pH 6.7.

The medium suitable for both first stage and second stage vegetative medium may be of the following composition: Baby oatmeal 20.0 g; sucrose 20.0 g; yeast 2.5 g; dried grain 5.0 g; $K_2HPO_4$ 1.0 g; Czepek's mineral stock 5.0 ml and deionized water to 1 liter, final pH 6.8.

The production medium may be of the following composition: Peanut meal 10.0 g; soluble meat peptone 5.0 g; sucrose 20.0 g; $KH_2PO_4$ 0.5 g; $K_2HPO_4$ 1.2 g; $MgSO_4 \bullet 7H_2O$ 0.25 g; tap water q.s. to 1 liter.

An alcohol solution of a cyclohexapeptide (IB) where E is stearoyl (tetrahydroechinocandin B) or palmitoyl (aculeacin A) is added to the production fermentation medium above-described and the deacylation monitored by assay against Candida albicans.

On completion of the deacylation, the fermentation broth containing the nucleus compound is filtered, passed through HP-20 resin to place the nucleus compound on the column and the compound recovered by elution with water/methanol (7/3) at a rate of 200-300 ml/minute. The eluate is monitored for the nucleus compound by treating with acid chloride and assaying for activity against Candida albicans. The eluate fractions showing activity are concentrated to obtain the nucleus compound (IBi).

The deacylated cyclopeptide compound thus obtained is purified by dissolving in water/acetonitrile/acetic acid/pyridine (96/2/1/1) and purified by reversed phase liquid chromatography (LICHROPREP RP-18) at a flow rate of about 60 milliliters per minute and monitoring the separation at 280 nm using a UV monitor. On the basis of the absorption at 280 nm, the fractions containing the desired compound are combined, concentrated under reduced pressure and employed for acylation or lyophilized for subsequent use.

The preparation of the deacylated cyclohexapeptide compound having the same nucleus from echinocandin type natural products is more fully described in U.S. 4,293,482. Similar preparations of other deacylated cyclohexapeptide compounds with similar nuclei may be found described in U.S. patents 4,173,629; 4,293,490; 4,299,763; 4,299,762; and 4,304,716.

The deacylated compounds may then be employed to produce novel and/or unusual acylated compounds.

The acylation of the compound of the deacylated nucleus, to produce a unique acyl derivative may be illustrated with a preparation of a compound in which E in formula (IB) is

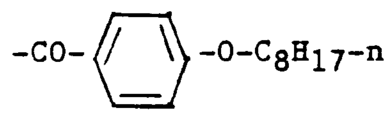

(Compound IBii). Such a compound would also be illustrated of a compound of formula (I) in which R is a radical defined under (C)(i).

In the acylation, the acyl group is preferably introduced by means of a trichlorophenyl ester. Thus, first, the trichlorophenyl ester may be prepared by treating the side chain acid with 2,4,5-trichlorophenol in the presence of a coupling agent such as N,N'-dicyclohexylcarbodiimide in an inert solvent such as methylene chloride. About 1.1 mole of the 2,4,5-trichlorphenol and 1.0 mole of the coupling agent is used for each mole of the alkoxybenzoic acid. The mixture is stirred at room temperature for 15 to 18 hours to obtain the ester which may be recovered by filtering the solid and evaporating the filtrate under reduced pressure, then recrystallizing the residue.

The ester thus prepared is added to a solution of the nucleus compound in dimethylformamide and stirred for about 18 hours and then the solvent evaporated off. The residue is washed and then chromatographed on silica gel using ethyl acetate-methanol (3/2) as eluant to obtain the desired octyloxybenzoyl derivative which may be represented in formula IB with the octyloxybenzoyl group as E. Such a compound may be named 1-[(4R,5R)-4,5-dihydroxy-$N^2$-[4-(octyloxy)benzoyl]-L-ornithine]echinocandin B (Compound IBii).

In addition to Compound IA, other preferred compounds include Compound IBii; echinocandin B (R', R'', R''', R'''' are OH; R''''' and R'''''' are $CH_3$; R is

$$-\overset{O}{\overset{\|}{C}}(CH_2)_4CH=CH(CH_2)_7CH_3;$$

tetrahydroechinocandin B (R', R'', R''' , R'''' are OH; R''''' and R'''''' are $CH_3$; R is

$$-\overset{O}{\overset{\|}{C}}(CH_2)_{16}CH_3);$$

O-methyltetrahydroechinocandin B (R', R'', and R'''' are OH; R''' is $-OCH_3$; R''''' and R'''''' are $CH_3$; R is

$$-\overset{O}{\overset{\|}{C}}(CH_2)_{16}CH_3);$$

and O-benzyltetrahydroechinocandin B (R', R'' and R'''' are OH, R''' is $-OCH_2C_6H_5$, R''''' and R'''''' are $CH_3$; R is

$$-\overset{O}{\overset{\|}{C}}(CH_2)_{16}CH_3).$$

The cyclohexapeptide compounds of Formula I of use in the present invention may be administered in therapeutically effective or anti-infective amounts to subjects infected with or immune compromised subjects susceptible to being infected with Pneumocystis carinii.

The efficacy of the cyclohexapeptide compounds for therapeutic and anti-infective purposes in immune-compromised patients may be determined first in studies on immunosuppressed rats and immunosuppressed mice.

In a representative study employing Compound IA in rats, nine male Sprague-Dawley rats weighing approximately 200 grams each, were immunosupressed by the addition of dexamethasone to the drinking water (2.0 mg/liter) for six weeks to induce the development of P. carinii infections. To enhance the infection the rats were also maintained on a low protein diet. At the beginning of the seventh week the rats were divided into three groups of three rats each. All three groups continued to receive dexamethasone in the drinking water and low protein diet throughout the remainder of the study. The rats in Group I were kept as untreated infected controls, those in Group II were injected intraperitoneally twice daily for two weeks with 0.5 ml of sterile $dH_2O$ containing 2 mg of Compound IA, and those in Group III were treated with trimethoprim-sulfamethoxazole (TMP-SMZ) in the drinking water (208 mg TMP and 1.040 g of SMZ/liter) for two weeks, a known treatment for P. carinii infections. Two rats died early in the experiment. One was from group II (rat 72A) and the other from group III (rat 75A). It was not determined whether they died from Pneumocystis pneumonia.

At the end of the two week treatment period (a total of eight weeks of immonosuppression) the animals were sacrificed and the lung tissue removed. The lungs from each animal were weighed, and then processed to determine the number of cysts and parasite nuclei for each animal. The results of this experiment are shown in Table I.

TABLE I

IN VIVO TESTING OF COMPOUND IA & TMP-SMZ FOR ACTIVITY AGAINST P. CARINII

| | RAT # | TOTAL CYSTS per lung | TOTAL CYSTS per gm lung | TOTAL NUCLEI per lung | TOTAL NUCLEI per gm lung | LUNG WT. (gms) | CYST SCORE |
|---|---|---|---|---|---|---|---|
| | 714 | $3.2x10^8$ | $1.0x10^8$ | $5.8x10^9$ | $1.8x10^9$ | 3.16 | 4 |
| GROUP I | 74A | $1.5x10^9$ | $4.5x10^8$ | $1.4x10^{10}$ | $4.2x10^9$ | 3.32 | 4+ |
| (control) | 74B | $7.5x10^8$ | $2.1x10^8$ | $1.4x10^{10}$ | $3.9x10^9$ | 3.56 | 4+ |
| GROUP II | 71B | N.D.* | N.D.* | $2.1x10^9$ | $7.4x10^8$ | 2.83 | 0 |
| (COMPOUND IA) | 72B | N.D.* | N.D.* | $1.0x10^9$ | $7.1x10^8$ | 1.40 | 0 |
| GROUP III | 73B | $6.0x10^6$ | $5.4x10^6$ | $2.0x10^8$ | $1.8x10^8$ | 1.11 | 1 |
| (TMP-SMZ) | 75B | $5.4x10^6$ | $2.3x10^6$ | $1.6x10^9$ | $7.0x10^8$ | 2.28 | 1 |

*N.D. = No cysts were detected in 50 fields, indicating that there were less than $2 x 10^5$ (limits of resolution).

These results demonstrate the Compound IA has efficacy against P. carinii infections. No cysts could be found in the lungs of either rat treated with Compound IA as compared to levels ranging from $3.2 x 10^8$ - $1.5 x 10^9$ in the control animals. There was also a reduction in the number of parasite nuclei ($1.0 - 2.1 x 10^9$) in these animals as compared to controls ($5.8 x 10^9$ - $1.4 x 10^{10}$). These results are superior to those seen in animals treated with TMP-SMZ, a known treatment for P. carinii infections, since the TMP-SMZ rats had detectable levels of P. carinii cysts.

A similar rat study was conducted to determine the antipneumocystis activity of 1-[(4R,5R)-4,5-dihydroxy-$N^2$-[4-(octyloxy)benzoyl]-L-ii ornithine]echinocandin B (Compound IBii). After 6 weeks of immunosuppression the rats were injected twice daily for 7 days with vehicle alone. Compound IBii (2 mg/kg) or Compound IA (2 mg/kg) during which time immunosuppression was continued. At the end of the treatment period the animals were sacrificed and the number of cysts per lung determined. Compound IA reduced the number of cysts by >99% while Compound IBii reduced the cyst counts by approximately 84% (compared to the vehicle control, 10% DMSO).

Studies were also carried out with mice and still other compounds within Formula I. Representatives of the method used in the studies carried out with mice and with other compounds is as follows:

Male C3H/HeN mice, weighing 22-24 grams each, were immunosuppressed by the addition of dexamethasone to the drinking water for six weeks to induce the development of P. carinii infections. To enhance the infections the mice were also maintained on a low protein diet. At the beginning of the seventh week, the mice were randomly divided into groups of 6. All groups continued to receive dexamethasone in the drinking water and low protein diet for the remainder of the study. Mice in each group were injected intraperitonially twice daily with 0.5 ml of a 10% DMSO solution as a vehicle control or the same cocktail containing drug to achieve a dose of 2 mg/kg. This procedure was continued for one week.

At the end of the treatment period (a total of seven weeks of immunosuppression) the animals were sacrificed and the lung tissue removed. The tissue was then processed to determine the number of cysts for each animal.

The results for the various compounds are seen in Table II.

TABLE II

| Compound | Dosage | % Reduction of Cysts |
|---|---|---|
| Tetrahydroechinocandin B (TEB) | 2mg/kg. | 99% |
| O-Methyl-TEB | 2mg/kg | >99% |
| O-Benzyl-TEB | 2mg/kg | 99% |
| Echinocandin B | 2mg/kg | >99% |
| Compound IA | 2mg/kg | >99% |

From the foregoing test results and from known dosage ranges for TMP-SMZ as applied to man, it is determined that generally from about 0.5 to about 20.0 mg/kg of body weight of the cyclohexapeptide anti-pneumocystis agent (Compound I) may be employed while considering the patients' health, weight, age and other factors which influence response to a drug as well as whether it is to be applied to a human patient or to an animal. These amounts, when expressed as doses suitable for human beings, are in the range of from about 35 mg to about 1500 mg daily preferably by parenteral administration.

The outstanding properties are most effectively utilized when the compound is formulated into novel pharmaceutical compositions with a pharmaceutically acceptable carrier according to conventional pharmaceutical compounding techniques.

The novel compositions contain at least 1% by weight of the active compound. In preparing the compositions, Compound I is intimately admixed with any of the usual pharmaceutically acceptable carrier.

The compositions include compositions suitable for oral, rectal, topical, parenteral (including subcutaneous, intramuscular, and intravenous), pulmonary (nasal or buccal inhalation), nasal administration, or insufflation.

The cyclohexapeptide anti-pneumocystis agent, Compound I, is preferably formulated into compositions for injection and may be presented in unit dosage form in ampoules or in multidose containers, if necessary with an added preservative. The compositions may also take such forms as suspensions, solutions or emulsions in oil or in vehicles such as 20/80 ethanol/propylene glycol or 20-35% polyethylene glycol 300 and may contain formulating agents such as suspending, stabilizing and/or dispersing agents. Alternatively, the active

ingredients may be in powder form for reconstituting with a suitable vehicle prior to administration or for use in insufflation.

The term "unit dosage form" as used in the specification and claims refer to physically discrete units, each unit containing a predetermined quantity of active ingredient calculated to produce the desired therapeutic effect in association with the pharmaceutical carrier. Examples of such unit dosage forms measured units in ampoules or in multidose containers and the like. A unit dosage of the present invention will generally contain from 100 to 1000 milligrams of the cyclohexapeptide anti-pneumocystis agent.

Any suitable route of administration may be employed for providing a patient with an effective dosage of a compound of Formula I. For example, oral, rectal, topical, parenteral, ocular, pulmonary, nasal, and the like may be employed. Dosage forms include tablets, troches, disperions, suspensions, solutions, capsules, creams, ointments, aerosols, powders for insufflations and the like.

The compositions include compositions suitable for oral, rectal, topical, parenteral (including subcutaneous, intramuscular, and intravenous), pulmonary (nasal or buccal inhalation), nasal administration, or insufflation.

For administration by inhalation, the compounds of the present invention are conveniently delivered in the form of an aerosol spray presentation from pressurized packs or nebulisers. The compounds may also be delivered as powders which may be formulated and the powder compositions may be inhaled with the aid of an insufflation powder inhaler device. The preferred delivery system for inhalation is a metered dose inhalation (MDI) aerosol, which may be formulated as a suspension or solution of Compound I in suitable propellants, such as fluorocarbons or hydrocarbons.

Because of the difficult solubility of the compound in pharmaceutically acceptable liquid carriers and because of desirability to directly treat lung and bronchi, aerosol administration is a preferred method of administration. Insufflation is also a desirable method, especially where infection may have spread to ears and other body cavities.

Alternatively, parenteral administration may be employed using drip intravenous administration. In such method, the drug may be solubilized in alcohol/propylene glycol or polyethylene glycol compositions. Intraperitoneal injection also may be employed.

The following examples illustrate the preparation of Compound IA and other lipoplilic cyclohexapeptide compound of Formula I and the compositions useful in the present invention of controlling Pneumocystis carinii.

PREPARATION OF COMPOUND IA

EXAMPLE A

Fermentation

A frozen culture in glycerol of Isolate 2 of Culture 8525-307P originally isolated from water, identifiable as Zalerion Arboricola, ATCC 20868, and maintained in the Merck culture collection was employed in the fermentation.

A 2 milliliter portion of the frozen culture was defrosted and aseptically transferred to a 250 milliliter unbaffled Erlenmeyer flask containing 54 milliliters of Medium 1. Medium 1, after inoculation, was incubated at 28°C with rotary agitation (220 rpm, 2" throw shaker) for three days. At the end of this period, 2.0 milliliters of the growth medium were aseptically transferred to each of several unbaffled 250 milliliter Erlenmeyer flasks containing Medium 1. The inoculated flasks were incubated at 28°C for 2 days.

12.5 milliliters of the mature seed broth were inoculated into five production flasks containing Medium 2, and incubated at 25°C for seven days under static conditions to obtain an antibiotic compound on the fermentation medium.

The media employed in the foregoing fermentation were:

MEDIUM 1 (KF Seed Medium)

| | |
|---|---|
| Corn Steep Liquor | 5 g |
| Tomato Paste | 40 g |
| Oat Flour | 10 g |
| Glucose | 10 g |
| Trace Elements Mix | 10 ml |
| Distilled Water | to 1000 ml |
| pH 6.8 | |

| | |
|---|---|
| Trace Elements Mix: | |
| $FeSO_4 \cdot 7H_2O$ | 1 g |
| $MnSO_4 \cdot 4H_2O$ | 1 g |
| $CuCl_2 \cdot 2H_2O$ | 25 mg |
| $CaCl_2$ | 100 mg |
| $H_3BO_3$ | 56 mg |
| $(NH_4)_6MoO_2 \cdot 4H_2O$ | 19 mg |
| $ZnSO_4 \cdot 7H_2O$ | 200 mg |
| Distilled Water | to 1000 ml |

MEDIUM 2 (F204 Solid Medium)

| | Amount/flask |
|---|---|
| Millet Base | 15 g |
| Yeast Extract | .5 g |
| Sodium tartrate | .1 g |
| Ferrous Sulfate Crystals | .01 g |
| Monosodium Glutamic Acid | .1 g |
| Corn Oil | .1 ml |

Isolation

Five hundred milliliters of methanol were added to each of the five 2-liter flasks of solid phase fermentation. The contents of the flask were then combined and stirred to extract methanol-soluble material and the mixture then filtered. The spent cake was stirred with an additional 2500 milliliters of methanol to further extract methanol soluble material and the mixture then filtered.

The filtrate and wash were combined and concentrated to 500 milliliters.

The aqueous methanolic concentrate thus obtained then was extracted with two 500 milliliter portions of ethyl acetate. The spent aqueous solution was placed onto a DIAION HP-20 column to adsorb the active material thereon and the latter then eluted therefrom with methanol. The eluates were combined with the previously obtained ethyl acetate extracts and the combined ethyl acetate solutions were concentrated to dryness. The residue was chromatographed on 200 milliliters of SEPHADEX LH-20 using 5:5:2 methylene chloride/hexane/methanol as eluant.

The fractions active as determined by Candida albicans were combined and chromatographed on 200 milliliters of silica gel (EM Science, KEISELGEL 60, 230-400 mesh) using a step gradient elution with ethyl acetate/methanol. The active fractions from this chromatogrphay were combined, concentrated and chromatographed on silica gel using a 75:25 ethyl acetate/methanol isocratic system.

The active portions from this chromatography were then combined and placed on 100 milliliters of SEPHADEX LH-20 using methanol as eluting solvent. The eluate, after vaporization of the solvent, yielded 95 milligrams of a purified compound. The compound was a white solid having a $^1$H NMR spectrum as seen in Figure 1.

EXAMPLE B

Fermentation

In a manner similar to that described in Example A, the contents of one frozen vial of ATCC 20868 from the Merck culture collection were defrosted and aseptically transferred to a 250 milliliter unbaffled flask containing 54 milliliters of KF medium (Medium 1) containing 0.4 percent agar. The modified Medium 1, after inoculation, was incubated at 28°C with 220 rpm agitation for 48 hours. At the end of this period, 10 milliliters of the growth medium was transferred to a 2-liter unbaffled flask containing 500 milliliters of KF medium containing 0.4 percent agar. After inoculation, the resulting medium was incubated for 24 hours at 28°C with 220 rpm agitation.

Twenty 2-liter flasks each containing 120 grams of Medium 2 and 120 milliliters of a stock solution consisting of

| | |
|---|---|
| Yeast extract | 5 parts by weight |
| Sodium tartrate | 1 part by weight |
| Ferrous sulfate crystals | 0.1 parts by weight |
| Monosodium glutamic acid | 1 part by weight |
| Corn oil | 1 part by weight |

were autoclaved for 20 minutes at 122°C and then reautoclaved with 80 milliliters of water for another 20 minutes at 122°C. The flasks were allowed to cool, then inoculated with 20 milliliters of seed medium prepared as above described, and the inoculated flasks incubated at 25°C under static conditions for 14 days.

Isolation

To each of nineteen 2-liter solid fermentation flasks was added 1 liter of methanol and the contents combined, stirred and filtered. The spent cake was extracted twice with 6 liters of methanol. The aqueous methanol filtrates were then concentrated and the concentrate extracted twice with 3 liters of ethyl acetate. The ethyl acetate extracts were combined, dried and concentrated to about 100 milliliters.

The concentrate was then coated on silica gel by adding 100 milliliters of methanol and 100 milliliters of silica gel thereto, intimately contacting the components and then removing the solvent on a rotary evaporator. The dried silica gel was then applied to a column of 500 milliliters of silica gel, the column washed with ethyl acetate to remove impurities and eluted with 9:1 ethyl acetate/methanol. The eluates containing antibiotic material testing positive against Candida albicans were recovered and combined.

The antibiotic rich cut from the silica gel chromatography was dissolved in 200 milliliters of 10:10:1 methylene chloride/hexane/methanol and the resulting solution combined with 40 milliliters of SEPHADEX LH-20 (which previously had been prepared by soaking overnight in methanol followed by washing twice with 200 milliliters of methylene chloride/hexane/methanol). After a few minutes, the supernatant was removed by filtration and the SEPHADEX LH-20 was washed with 200 milliliter of methylene chloride/hexane/methanol and then filtered. The filtrates were found not to contain the active constituent, and were discarded. The active constituent which had partitioned into the dextran SEPHADEX LH-20 beads was extracted therefrom by washing twice with 200 milliliters of methanol, and these methanol washes were combined and concentrated.

The methanol concentrate was next applied to 200 milliliters of silica gel and eluted with 75:25 ethyl acetate/methanol and the eluates combined and the solvent vaporized to obtain Compound IA. Compound IA is a white powder having a decomposition point of 206-214°C.

PREPARATION OF ECHINOCANDIN B DERIVATIVES

EXAMPLE C

Preparation of Echinocandin B

A. Fermentation

Culture MF 5100 Emericella nidulans ATCC 20956 from the Merck culture collection was used for seed train development as follows: One frozen vial (about 2.5 ml) of MF 5100 was used to inoculate 54 mL of KF medium and cultured in 250 ml unbaffled Erlenmeyer flasks at 28°C and 220 rpm for 72 hours to obtain "B" stage culture. Five mL of "B" stage culture was used to inoculate 500 mL of KF medium in 2 liter unbaffled Erlenmeyer flask and incubated at 28°C and 220 rpm for 48 hours to obtain "C" stage cultures. Four "C" stage flasks were used to inoculate 160 liters of KF medium with trace element mix #2 in a "D" stage (300 liter stirred fermenter).

The three stages of seed train were used for "E" scale fermentation in an 800 liter fermenter. The fermentation was carried out for 23 hours at 28°C, back pressure 0.7 kg/cm$^2$, aeration with 0.300m (53 liters per minute) and agitation of 100 rpm.

The production "E" stage was inoculated with 5% seed from "D" stage in 5700 liters of a production medium of the following composition:

| | |
|---|---|
| Glycerol | 85 g/L |
| Pectin | 10 g/L |
| Peanut Meal | 4 g/L |
| Peptonized Meal | 4 g/L |
| Peptonized Milk | 4 g/L |
| Tomato Paste | 4 g/L |
| Corn Steep Liquor | 4 g/L |
| Lard Water | 4 g/L |
| Glycine | 2 g/L |
| Potassium phosphate monobasic | 2 g/L |
| Polyglycol P-2000 | 1 mL/L |

The medium was sterilized for 25 minutes at 123°C and the fermentation then carried out at temperature of 28°C, positive pressure of about 0.8 kg/cm$^2$, aeration 6000 liters per minute, agitation speed of 120 rpm; pH in the range 5.5-6.5 with dissolved oxygen maintained above 30 percent for about 36 hours.

B. Isolation

The whole broth from the above fermentation containing 355 grams of echinocandin B by HPLC assay (against reference standard echinocandin B) was extracted overnight with an equal volume of methanol. The diluted broth was centrifuged to remove broth solids and the centrifugate adsorbed on a 450 liter SP 207 column which had been equilibrated with 50:50 methanol/water. After a 1000 liter water wash and a 1000 liter 50:50 methanol/water wash, the column was eluted with methanol and the eluate concentrated to 189 liters and partitioned with methylene chloride. The aqueous methanol layer was adjusted to 50:50 methanol/water by the addition of water and absorbed on a 100 liter HP-20 column prewashed with methanol and pre-equilibrated with 50:50 methanol/water. After a 50:50 methanol/water wash, the column was eluted with 80:20 methanol/water. The 80:20 eluate was adjusted to 50:50 with water, and then was adsorbed on a 27 liter SP207 column, preequilibrated as before. The column was then eluted with methanol.

A 19 g aliquot of SP 207 eluate was diluted to 50:50 methanol/water with water and adsorbed on a 3.9 liter Amicon preparative C-18 column. A gradient of acetonitrile/water (25:75) to 100 percent acetonitrile over a 75 minute period was used to elute the compound. Selected fraction after concentration and lyophilization from acetonitrile/water provided 16 grams of Echinocandin B of 87-91 percent purity.

The identity and purity of the foregoing echinocandin B was determined by HPLC and spectral comparison with a sample of Echinocandin B, of the properties below, previously prepared and purified, and its identity established by comparison with a published structure of Echinocandin B.

EXAMPLE D

Preparation of Tetrahydroechinocandin B

5.0 Grams of Echinocandin B (EB) of about 87 percent purity (HPLC) was dissolved in 150 milliliters of absolute ethanol and was added to 10% Pd-C in 80 milliliters of ethanol which had been prereduced under 1 atmosphere of hydrogen for 20 minutes. After the addition, the mixture was stirred rapidly under 1 atmosphere of hydrogen for 4 hours. Analysis by HPLC using $CH_3CN/H_2O$ (81/19), on a C8 ZORBAX column at flow rate 1 ml/min at 30°C showed that the reaction was virtually complete at this time.

The reduction mixture was filtered through a Celite pad, the pad washed with ethanol and the mixture was concentrated in vacuo to obtain a solid. The solid was purified in ten 0.5 gram batches dissolved in 5 milliliters of $CH_3CN/H_2O$ (81/19). The HPLC was carried out on C8 ZORBAX 1 inch column and eluted with 80% methanol/20% water at a flow rate of 15 ml/min. A total yield of 3.15 grams (79%) of tetrahydroechinocandin B was obtained.

EXAMPLE E

Preparation of O-Methyltetrahydroechinocandin B

55 Milligrams of tetrahydroechinocandin B was dissolved in 4 milliliters of methanol. To the solution was added 4.0 milligrams of p-toluenesulfonic acid and the mixture stirred at room temperature for 3 hours. HPLC analysis indicated that the reaction was complete at this time.

The reaction mixture was quenched with 1N sodium bicarbonate solution and washed twice with saturated sodium chloride solution and then diluted with 200 milliliters of isopropanol/chloroform (2/7).

The organic layers were dried over sodium sulfate, filtered and the solvent vaporized to obtain a near white solid as residue. The latter was purified first with methanol, and then with $H_2O/CH_3CN$ (2:3) and chromatographing by MPLC in $H_2O/CH_3CN$ (2:3) on a C8 LOBAR size A column in 4 milliliter fractions. The compound was obtained in a yield of 42.4 mg. (76%)

EXAMPLE F

Preparation of O-Benzyltetrahydroechinocandin B

50 Milligrams of tetrahydroechinocandin B was suspended in 1 milliliter of tetrahydrofuran. To it was added 97 milliliters of anhydrous benzyl alcohol and the resulting mixture stirred until all was in complete solution. To the resulting solution was added 1 mg. of p-toluenesulfonic acid monohydrate and the mixture then stirred at room temperature for 3 hours whereupon a HPLC analysis showed that the reaction was complete. The reaction mixture was diluted with 1N sodium bicarbonate solution and then with isopropyl alcohol/chloroform (3:7). The organic solution was washed twice with water, dried and evaporated to recover the product as residue.

The following examples illustrate novel compositions useful in the practice of the present invention.

Aerosol compositions may be prepared having the following formulations:

EXAMPLE I

| | Per Canister |
|---|---|
| Compound Ia | 24 mg |
| Lecithin, NF Liquid Concentrate | 1.2 mg |
| Trichlorofluoromethane | 4.025 g |
| Dichlorodifluoromethane | 12.15 g |

EXAMPLE II

| | Per Canister |
|---|---|
| Echinocandin B | 24 mg |
| Oleic acid | 1.2 mg |
| Trichlorofluoromethane | 4.025 g |
| Dichlorodifluoromethane | 12.15 g |

EXAMPLE III

| | Per Canister |
|---|---|
| O-Methyltetrahydroechinocandin B | 24 mg |
| Sorbitan trioleate | 1.2 mg |
| Trichlorofluoromethane | 4.025 g |
| Dichlorodifluoromethane | 12.15 g |

An injectible suspension (1M) may be prepared as follows:

EXAMPLE IV

| | mg/ml |
|---|---|
| Compound Ia | 10 |
| Methylcellulose | 5.0 |
| Tween 80 | 0.5 |
| Benzyl alcohol | 9.0 |
| Benzalkonium chloride | 1.0 |
| Water to 1 ml. | |

General Information

For Echinocandin B

Emericella nidulans (a later growth stage of Aspergillus nidulans, established as a producing organism for Echinocandin B) and maintained as MF 5100 in the Merck culture collection was employed in the fermentation. A culture was deposited with the American Type Culture Collection under the Budapest Treaty and assigned ATCC No. 20956.

Echinocandin B, prepared as described in Example C was compared for percent purity against previously prepared Echinocandin B having the following MS and NMR spectra and identified by comparison with published structure for Echinocandin B.

Mass Spectral Data: FAB mass spectra were recorded on a VG 20-253 mass spectrometer. A matrix of dithiothreitol-dithiocrythritol-LiI was used and M+Li was observed at m/z 1066 corresponding to the molecular weight 1059.

$^1$H NMR Spectrum: The spectrum, Fig. 2, was recorded in $CD_3OD$ on a Varian XL-300 NMR Spectrometer at 300 MHz. Chemical shifts are shown in ppm relative to tetramethylsilane (TMS) at zero ppm using the solvent peak at 3.30 ppm as internal reference.

$^{13}$C NMR Data: The spectrum was recorded in $CD_3OD$ at 24°C on a Varian XL-400 NMR spectrometer at 100 MHz. Chemical shifts are given in ppm relative to tetramethylsilane at zero ppm using the solvent peak at 49.0 ppm as internal reference.

$^{13}$C Chemical Shifts ($CD_3OD$, 100 MHz): 11.28, 14.43, 19.66, 20.04, 23.62, 26.54, 27.05, 28.15, 28.20, 30.27(x2), 30.42, 30.46, 30.78, 32.65, 35.08, 36.82, 38.60, 39.06, 51.67, 52.94, 56.32, 56.84, 57.19, 58.61, 62.46, 68.33, 69.59, 69.70, 70.87, 71.29, 74.60, 75.52, 75.77, 76.94, 116.21(x2), 129.06(x2), 129.60(x2), 130.94, 130.96, 133.09, 158.46, 169.97, 172.48, 172.51, 172.79, 173.53, 174.35, 176.19.

## Claims

### Claims for the following Contracting States : AT, BE, CH, DE, FR, GB, IT, LI, LU, NL, SE

1. The use of a cyclohexapeptide compound represented by the formula

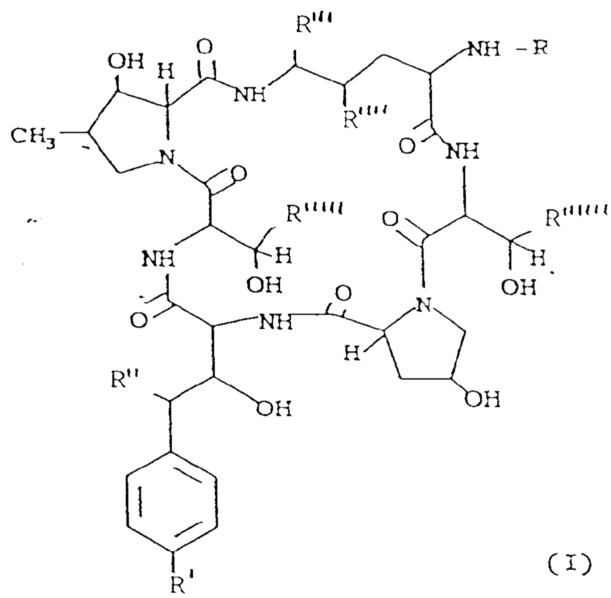

(I)

wherein
R' is H or OH;
R'' is H or OH;
R''' is H, OH, -O-alkyl($C_1$-$C_6$), -O-benzyl, or

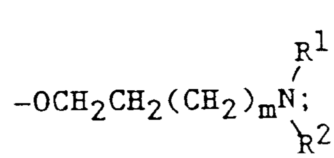

wherein $R^1$ is H, $C_1$-$C_{12}$ alkyl, $C_3$-$C_7$ cycloalkyl, hydroxyethyl, $C_{1-6}$ alkoxyethyl, furylmethyl, tetrahydrofurylmethyl, phenyl, phenylalkyl wherein the phenyl may be substituted with halo, hydroxy, $C_{1-6}$ alKyl and $C_{1-6}$ alkoxy;

$R^2$ is hydrogen or $C_{1-6}$ alKyl; or $R^1$ and $R^2$ together form $-(CH_2)_2Q(CH_2)_2-$ wherein Q is $-CH_2-$,-NH- or -N-($C_{1-6}$)-alkyl;

and m is from 0 to 4;
R'''' is H or OH;
R''''' is H, $CH_3$ or $CH_2CONH_2$;
R'''''' is H or $CH_3$; and
R is
(A)

$$-\overset{\overset{\displaystyle O}{\|}}{C}-\text{alkyl}$$

from 6 to 24 carbon atoms
(B)

$$-\overset{\overset{\displaystyle O}{\|}}{C}-\text{alkenyl}$$

from 6 to 24 carbon atoms
(C) when R′, R″, R‴ and R⁗ are OH, R′′′′′ is $CH_3$ then

(i)

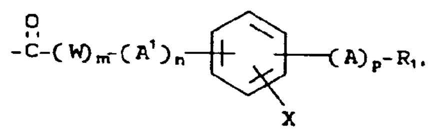

(ii)

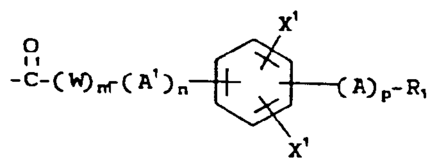

or

(iii)

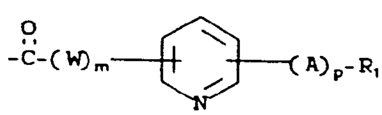

wherein A is divalent oxygen, sulfur, sulfinyl or sulfonyl;

$A^1$ is divalent oxygen, sulfur, sulfinyl, sulfonyl or -NH-;

X is hydrogen, chloro, bromo, iodo, nitro, $C_1$-$C_3$ alkyl, hydroxy, $C_1$-$C_3$ alkoxy, mercapto, $C_1$-$C_3$ alkylthio, carbamyl or $C_1$-$C_3$ alkylcarbamyl;

$X^1$ is chloro, bromo or iodo,

$R^1$ is hydrogen, $C_1$-$C_{18}$alkyl or $C_2$-$C_{18}$alkenyl;

W is $C_1$-$C_{10}$alkylene or $C_2$-$C_{10}$alkenylene;

m,n and p are 0 or 1, but if m is 0, n must be 0;

provided that the sum of the carbon atoms in the $R_1$ and W groups must be greater than 4 but cannot exceed 21; that when X is mercapto, A and $A^1$ cannot be sulfinyl or sulfonyl; and that when A and $A^1$ are sulfinyl or sulfonyl, they must be in equal oxidation states;

(D) when R′ is OH, and R", R‴ and R⁗ are H, then

$$-Y-\overset{\overset{\displaystyle O}{\|}}{C}-R_2$$

wherein Y is a divalent aminoacyl radical of the formula

a)

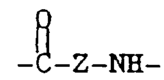

wherein Z is $C_1$-$C_{10}$ alkylene or $C_5$-$C_6$ cycloalkylene

b)

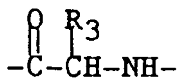

wherein $R_3$ is hydroxymethyl, hydroxyethyl, mercaptomethyl, mercaptoethyl, methylthioethyl, 2-thienyl, 3-indolomethyl, phenyl, benzyl, halophenyl, halobenzyl, $C_1$-$C_3$ alkylphenyl, $C_1$-$C_3$ alkylbenzyl, $C_1$-$C_3$ alkylthiophenyl, $C_1$-$C_3$ alkylthiobenzyl, carbamylphenyl, carbamylbenzyl, $C_1$-$C_3$ alkylcarbamylphenyl or $C_1$-$C_3$ alkylcarbamylbenzyl;

(c)

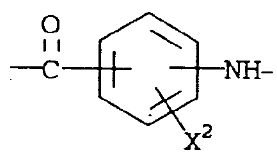

wherein $X^2$ is hydrogen or halo; for the preparation of a medicament, useful for the treatment of or for the prevention of Pneumocystis carinii infections.

2. The use according to Claim 1 wherein the cyclohexapeptide compound is administered at a dose of from 0.5 to 20.0 mg/kg of mammalian body weight.

3. The use according to Claim 1 wherein the cyclohexapeptide compound is administered to a patient with an impaired immune system infected with Pneumocystis carinii.

4. The use according to Claim 1 wherein the cyclohexapeptide compound is administered to a patient infected with Pneumocystis carinii and exhibits AIDS.

5. A composition useful for the treatment of Pneumocystis carinii infections in mammals and adapted for administration by inhalation, comprising a pharmaceutically acceptable carrier and a cyclohexapeptide compound having the formula

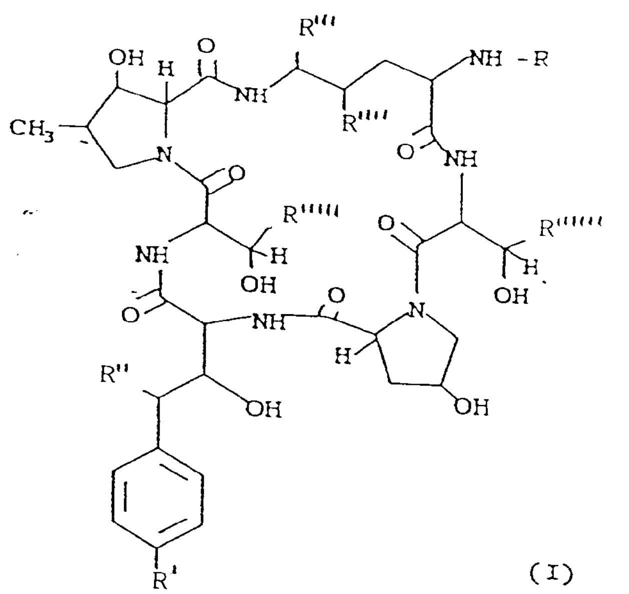

(I)

wherein

R′ is H or OH;
R″ is H or OH;
R‴ is H, OH, -O-alkyl($C_1$-$C_6$), -O-benzyl, or

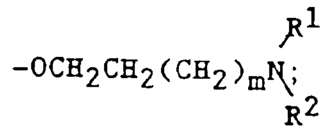

wherein $R^1$ is H, $C_1$-$C_{12}$ alkyl, $C_3$-$C_7$ cycloalkyl, hydroxyethyl, $C_{1-6}$ alkoxyethyl, furylmethyl, tetrahydrofurylmethyl, phenyl, phenylalkyl wherein the phenyl may be substituted with halo, hydroxy, $C_{1-6}$ alkyl and $C_{1-6}$ alkoxy; $R^2$ is hydrogen or $C_{1-6}$ alkyl; or $R^1$ and $R^2$ together form $-(CH_2)_2Q(CH_2)_2-$ wherein Q is $-CH_2-$,-NH- or -N-($C_{1-6}$)-alkyl; and m is from 0 to 4;
R⁗ is H or OH;
R‴″ is H, $CH_3$ or $CH_2CONH_2$; and
R‴‴ is H or $CH_3$; and
R is
(A)

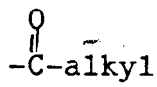

from 6 to 24 carbon atoms
(B)

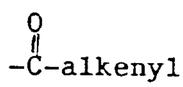

from 6 to 24 carbon atoms
(C) when R′, R″, R‴ and R⁗ are OH, R‴″ is $CH_3$ then
(i)

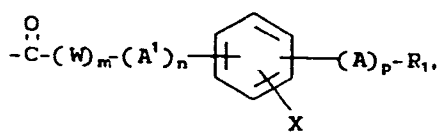

(ii)

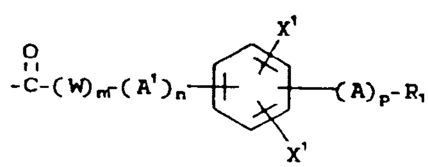

or
(iii)

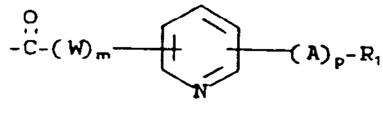

wherein A is divalent oxygen, sulfur, sulfinyl or sulfonyl;

$A^1$ is divalent oxygen, sulfur, sulfinyl, sulfonyl or -NH-;

X is hydrogen, chloro, bromo, iodo, nitro, $C_1$-$C_3$ alkyl, hydroxy, $C_1$-$C_3$ alkoxy, mercapto, $C_1$-$C_3$ alkylthio, carbamyl or $C_1$-$C_3$ alkylcarbamyl;

$X^1$ is chloro, bromo or iodo,

$R^1$ is hydrogen, $C_1$-$C_{18}$ alkyl or $C_2$-$C_{18}$ alkenyl;

W is $C_1$-$C_{10}$alkylene or $C_2$-$C_{10}$ alkenylene;

m,n and p are 0 or 1, but if m is 0, n must be 0;

provided that the sum of the carbon atoms in the $R_1$ and W groups must be greater than 4 but cannot exceed 21; that when X is mercapto A and $A^1$ cannot be sulfinyl or sulfonyl; and that when A and $A^1$ are sulfinyl or sulfonyl, they must be in equal oxidation states;

(D) when R′ is OH, and R″, R‴ and R″″ are H, then

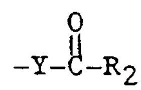

wherein Y is a divalent aminoacyl radical of the formula

a)

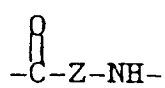

wherein Z is $C_1$-$C_{10}$ alkylene or $C_5$-$C_6$ cycloalkylene

b)

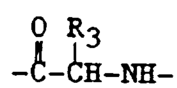

wherein $R_3$ is hydroxyethyl, hydroxyethyl, mercaptomethyl, mercaptoethyl, methylthioethyl, 2-thienyl, 3-indolomethyl, phenyl, benzyl, halophenyl, halobenzyl, $C_1$-$C_3$ alkylphenyl, $C_1$-$C_3$ alkylbenzyl, $C_1$-$C_3$ alkylthiophenyl, $C_1$-$C_3$ alkylthiobenzyl, carbamylphenyl, carbamylbenzyl, $C_1$-$C_3$ alkylcarbamylphenyl or $C_1$-$C_3$ alkylcarbamylbenzyl;

(c)

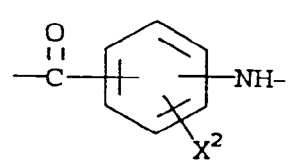

wherein $X^2$ is hydrogen or halo.

6. The use according to Claim 1 wherein the compound is that having the formula

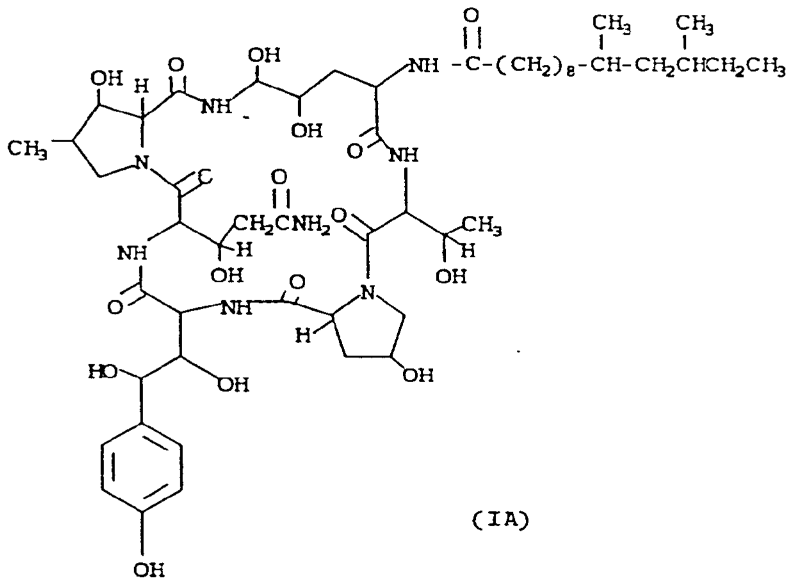

(IA)

7. The use according to Claim 1 wherein the compound is 1-[(4R,5R)-4,5-dihydroxy-$N^2$-(10,12-dimethyl-1-oxotetradecyl)-L-ornithine-5-(threo-3-hydroxy-1-glutamine)echinocandin B.

8. The use, according to Claim 1 wherein the compound is 1-[(4R,5R)-4,5-dihydroxy-$N^2$-[4-(octyloxy)benzoyl]-L-ornithine]echinocandin B.

9. The use according to Claim 1 wherein the compound is echinocandin B.

10. The use according to Claim 1 wherein the compound is tetrahydroechinocandin B.

11. The use according to Claim 1 wherein the compound is O-methyltetrahydroechinocandin B.

12. The use according to Claim 1 wherein the compound is O-benzyltetrahydroechinocandin B.

**Claims for the following Contracting State : ES**

1. A process for the preparation of a composition useful for the treatment of Pneumocystis carinii infections in mammals and adapted for administration by inhalation, which comprises admixing a pharmaceutically acceptable carrier with a cyclohexapeptide compound having the formula

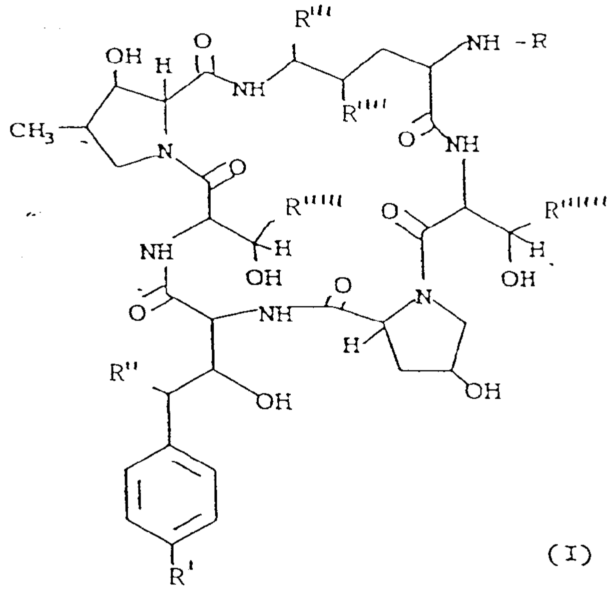

(I)

wherein

R′ is H or OH;

R″ is H or OH;

R‴ is H, OH, -O-alkyl($C_1$-$C_6$), -O-benzyl, or

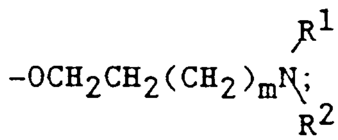

wherein $R^1$ is H, $C_1$-$C_{12}$ alkyl, $C_3$-$C_7$ cycloalkyl, hydroxyethyl, $C_{1-6}$ alkoxyethyl, furylmethyl, tetrahydrofurylmethyl, phenyl, phenylalkyl wherein the phenyl may be substituted with halo, hydroxy, $C_{1-6}$ alkyl and $C_{1-6}$ alkoxy; $R^2$ is hydrogen or $C_{1-6}$ alkyl; or $R^1$ and $R^2$ together form $-(CH_2)_2Q(CH_2)_2-$ wherein Q is $-CH_2-$, -NH- or -N-($C_{1-6}$)-alkyl; and m is from 0 to 4;

R'''' is H or OH;

R''''' is H, $CH_3$ or $CH_2CONH_2$; and

R'''''' is H or $CH_3$; and

R is

(A)

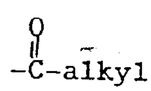

from 6 to 24 carbon atoms

(B)

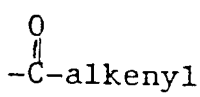

from 6 to 24 carbon atoms

(C) when R′, R″, R‴ and R'''' are OH R''''' is $CH_3$ then

(i)

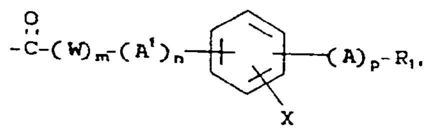

(ii)

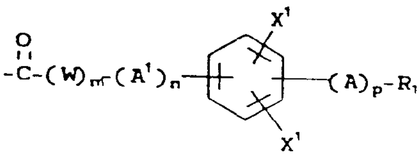

or
(iii)

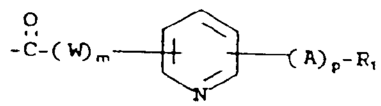

wherein A is divalent oxygen, sulfur, sulfinyl or sulfonyl;

$A^1$ is divalent oxygen, sulfur, sulfinyl, sulfonyl or -NH-;

X is hydrogen, chloro, bromo, iodo, nitro, $C_1$-$C_3$ alkyl, hydroxy, $C_1$-$C_3$ alkoxy, mercapto, $C_1$-$C_3$ alkylthio, carbamyl or $C_1$-$C_3$ alkylcarbamyl;

$X^1$ is chloro, bromo or iodo,

$R^1$ is hydrogen, $C_1$-$C_{18}$ alkyl or $C_2$-$C_{18}$ alkenyl;

W is $C_1$-$C_{10}$alkylene or $C_2$-$C_{10}$ alkenylene;

m,n and p are 0 or 1, but if m is 0, n must be 0;

provided that the sum of the carbon atoms in the $R_1$ and W groups must be greater than 4 but cannot exceed 21; that when X is mercapto A and $A^1$ cannot be sulfinyl or sulfonyl; and that when A and $A^1$ are sulfinyl or sulfonyl, they must be in equal oxidation states;

(D) when R′ is OH, and R″, R‴ and R″″ are H, then

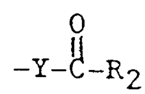

wherein Y is a divalent aminoacyl radical of the formula

a)

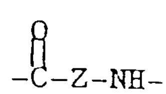

wherein Z is $C_1$-$C_{10}$ alkylene or $C_5$-$C_6$ cycloalkylene

b)

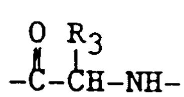

wherein $R_3$ is hydroxyethyl, hydroxyethyl, mercaptomethyl, mercaptoethyl, methylthioethyl, 2-thienyl, 3-indolomethyl, phenyl, benzyl, halophenyl, halobenzyl, $C_1$-$C_3$ alkylphenyl, $C_1$-$C_3$ alkylbenzyl, $C_1$-$C_3$ alkylthiophenyl, $C_1$-$C_3$ alkylthiobenzyl, carbamylphenyl, carbamylbenzyl, $C_1$-$C_3$ alkylcarba-

mylphenyl or $C_1$-$C_3$ alkylcarbamylbenzyl;
(c)

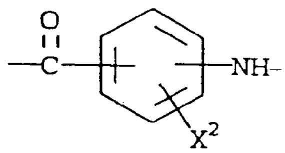

wherein $X^2$ is hydrogen or halo.

2. A process according to Claim 1 wherein the compound is that having the formula

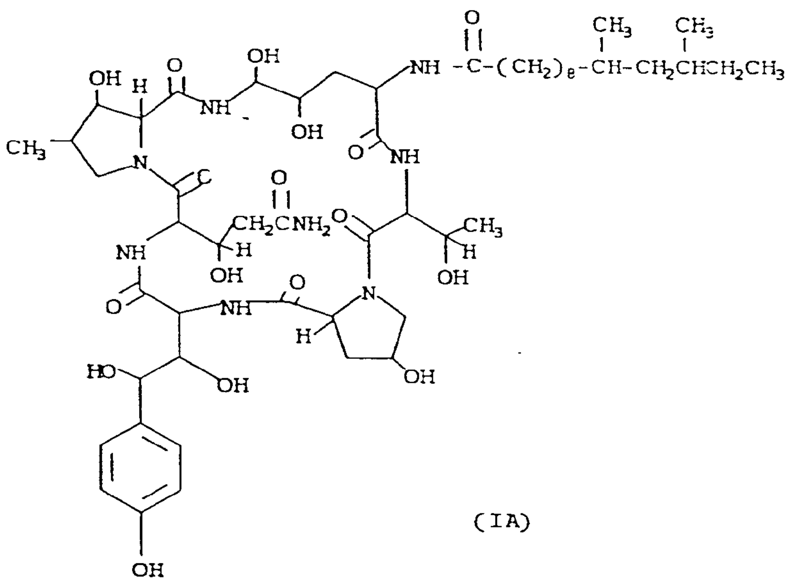

(IA)

3. A process according to Claim 1 wherein the compound is 1-[(4R,5R)-4,5-dihydroxy-$N^2$-(10,12-dimethyl-1-oxotetradecyl)-L-ornithine-5-(threo-3-hydroxy-1-glutamine)echinocandin B.

4. A process according to Claim 1 wherein the compound is 1-[(4R,5R)-4,5-dihydroxy-$N^2$-[4-(octyloxy)benzoyl]-L-ornithine]echinocandin B.

5. A process according to Claim 1 wherein the compound is echinocandin B.

6. A process according to Claim 1 wherein the compound is tetrahydroechinocandin B.

7. A process according to Claim 1 wherein the compound is O-methyltetrahydroechinocandin B.

8. A process according to Claim 1 wherein the compound is O-benzyltetrahydroechinocandin B.

**Patentansprüche**

**Patentansprüche für folgende Vertragsstaaten : AT, BE, CH, DE, FR, GB, IT, LI, LU, NL, SE**

1. Verwendung einer Cyclohexapeptidverbindung, dargestellt durch die Formel

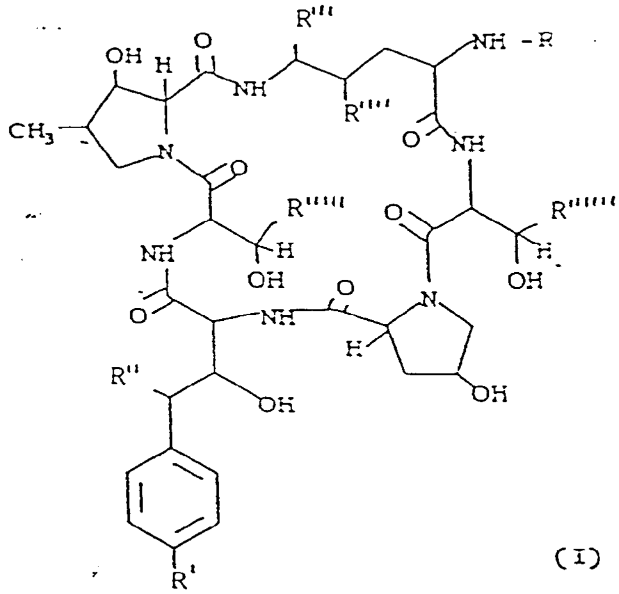

(I)

in der

R' H oder OH;

R'' H oder OH;

R''' H, OH, -O-Alkyl($C_1$-$C_6$), -O-Benzyl oder

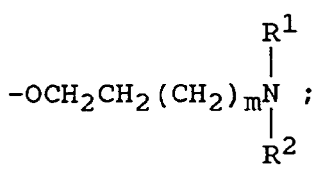

wobei $R^1$ H, $C_1$-$C_{12}$ Alkyl, $C_3$-$C_7$ Cycloalkyl, Hydroxyethyl, $C_{1-6}$ Alkoxyalkyl, Furylmethyl, Tetrahydrofurylmethyl, Phenyl, Phenylalkyl, worin der Phenylrest durch Halogen, Hydroxy, Cl-6 Alkyl oder $C_{1-6}$ Alkoxy substituiert sein kann, $R^2$ H oder $C_{1-6}$ Alkyl oder

$R^1$ und $R^2$ zusammen den Rest $-(CH_2)_2Q(CH_2)_2-$, in dem Q $-CH_2-$, -NH- oder -N-($C_{1-6}$)Alkyl ist, darstellen und m 0 bis 4 ist;

R'''' H oder OH;

R''''' H, $CH_3$ oder $CH_2CONH_2$;

R'''''' H oder $CH_3$ bedeuten und in der

R eine der folgenden Bedeutungen aufweist:

(A)

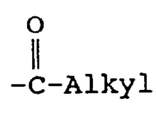

mit 6 bis 24 Kohlenstoffatomen,

(B)

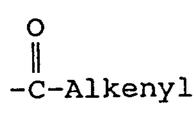

mit 6 bis 24 Kohlensatoffatomen,

(C) falls R', R'', R''' und R'''' OH sind und R''''' $CH_3$ ist, einen der Reste

(i)

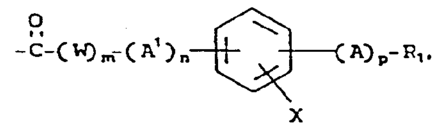

(ii)

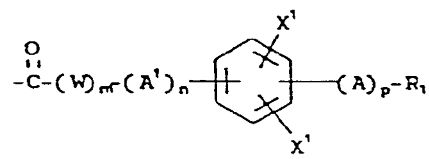

oder

(iii)

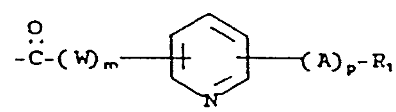

in denen A bivalenten Sauerstoff, Schwefel, Sulfinyl oder Sulfonyl;

$A^1$ bivalenten Sauerstoff, Schwefel, Sulfinyl, Sulfonyl oder -NH-;

X Wasserstoff, Chlor, Brom, Jod, Nitro, $C_1$-$C_3$ Alkyl, Hydroxy, $C_1$-$C_3$ Alkoxy, Mercapto, $C_1$-$C_3$ Alkylthio, Carbamoyl oder $C_{1-3}$ Alkylcarbamoyl;

$X^1$ Chlor, Brom oder Jod;

$R^1$ Wasserstoff, $C_1$-$C_{18}$ Alkyl oder $C_2$-$C_{18}$ Alkenyl;

und W $C_1$-$C_{10}$ Alkylen oder $C_2$-$C_{10}$ Alkenylen darstellen und

m, n und p 0 oder 1 sind, wobei jedoch, wenn m 0 ist, n auch 0 sein muß;

vorausgesetzt daß die Summe der Kohlenstoffatome in den Resten $R^1$ und W größer als 4 ist und 21 nicht überschreitet, daß A und $A^1$ nicht Sulfinyl oder Sulfonyl sein können, wenn X Mercapto bedeutet, und daß, wenn A und $A^1$ Sulfinyl oder Sulfonyl bedeuten, sie auf der gleichen Oxidationsstufe sein müssen;

(D) falls R′ OH ist und R″, R‴ und R″″ H sind, dann

$$-Y-\overset{\overset{\displaystyle O}{\|}}{C}-R_2$$

wobei Y ein bivalentes Aminoacylradikal der Formeln

a)

$$-\overset{\overset{\displaystyle O}{\|}}{C}-Z-NH-$$

worin Z $C_1$-$C_{10}$ Alkylen oder $C_5$-$C_6$ Cycloalkylen ist,

b)

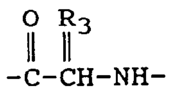

worin $R_3$ Hydroxymethyl, Hydroxyethyl, Mercaptomethyl, Mercaptoethyl, Methylthioethyl, 2-Thienyl, 3-Indolmethyl, Phenyl, Benzyl, Halogenphenyl, Halogenbenzyl, $C_1$-$C_3$ Alkylphenyl, $C_1$-$C_3$ Alkylbenzyl, $C_1$-$C_3$ Alkylthiophenyl, $C_1$-$C_3$ Alkylthiobenzyl, Carbamoylphenyl, Carbamoylbenzyl, $C_1$-$C_3$ Alkylcarbamoylphenyl oder $C_1$-$C_3$ Alkylcarbamoylbenzyl ist; und

c)

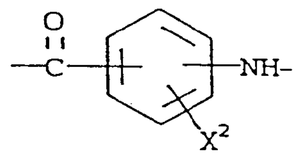

worin $X^2$ Wasserstoff oder Halogen ist; darstellt,

für die Herstellung eines Arzneimittels, welches für die Behandlung oder für die Vorbeugung von Infektionen durch Pneumocystis carinii verwendbar ist.

2. Verwendung gemäß Anspruch 1, wobei die Cyclohexapeptidverbindung in einer Dosis von 0,5 bis 20,0 mg/kg Säugetier verabreicht wird.

3. Verwendung gemäß Anspruch 1, wobei die Cyclohexapeptidverbindung einem mit Pneumocystis carinii infizierten Patienten mit geschwächtem Immunsystem verabreicht wird.

4. Verwendung gemäß Anspruch 1, wobei die Cyclohexapeptidverbindung einem mit Pneumocystis carinii infizierten, an AIDS leidenden Patienten verabreicht wird.

5. Eine Zusammensetzung, die für die Behandlung von Infektionen durch Pneumocystis carinii bei Säugetieren verwendbar und für die Verabreichung durch Inhalation angepaßt ist, umfassend einen pharmazeutisch annehmbaren Träger und eine Cyclohexapeptidverbindung mit der Formel

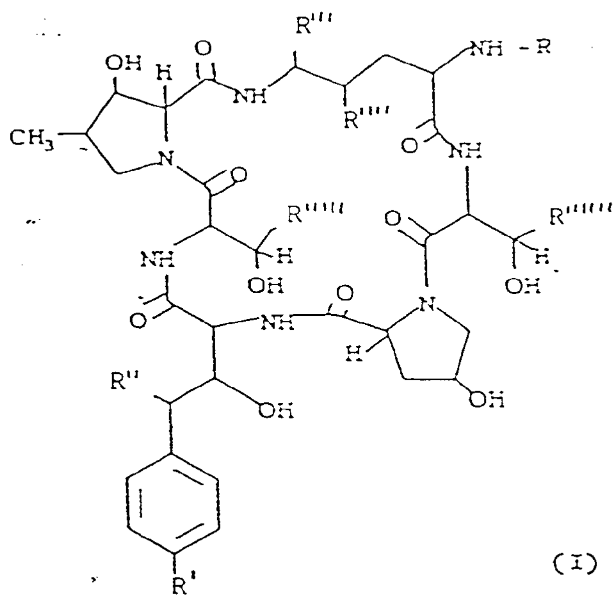

(I)

in der

R′ H oder OH;

R″ H oder OH;

R‴ H, OH, -O-Alkyl($C_1$-$C_6$), -O-Benzyl oder

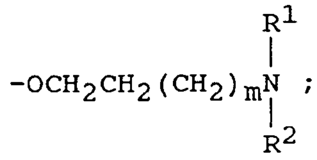

wobei $R^1$ H, $C_1$-$C_{12}$ Alkyl, $C_3$-$C_7$ Cycloalkyl, Hydroxyethyl, $C_{1-6}$ Alkoxyalkyl, Furylmethyl, Tetrahydrofurylmethyl, Phenyl, Phenylalkyl, worin der Phenylrest durch Halogen, Hydroxy, Cl-6 Alkyl oder $C_{1-6}$ Alkoxy substituiert sein kann,

$R^2$ H oder $C_{1-6}$ Alkyl oder

$R^1$ und $R^2$ zusammen den Rest $-(CH_2)_2Q(CH_2)_2-$, in dem Q $-CH_2-$, -NH- oder -N-($C_{1-6}$)Alkyl ist, darstellen und m eine ganze Zahl von 0 bis 4 ist;

R'''' H oder OH;

R''''' H, $CH_3$ oder $CH_2CONH_2$;

R'''''' H oder $CH_3$ bedeuten und in der R eine der folgenden Bedeutungen aufweist:

(A)

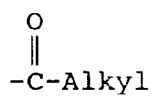

mit 6 bis 24 Kohlenstoffatomen,

(B)

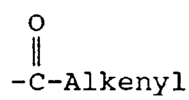

mit 6 bis 24 Kohlensatoffatomen,

(C) falls R', R'', R''' und R'''' OH sind und R''''' $CH_3$ ist, einen der Reste

(i)

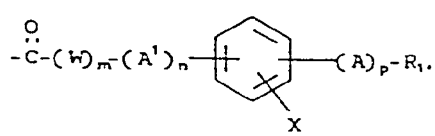

(ii)

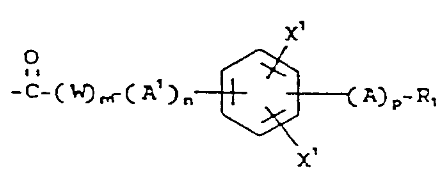

oder

(iii)

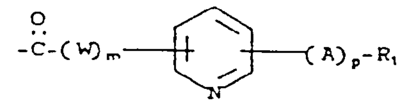

in denen A bivalenten Sauerstoff, Schwefel, Sulfinyl oder Sulfonyl;

$A^1$ bivalenten Sauerstoff, Schwefel, Sulfinyl, Sulfonyl oder -NH-;

X Wasserstoff, Chlor, Brom, Jod, Nitro, $C_1$-$C_3$ Alkyl, Hydroxy, $C_1$-$C_3$ Alkoxy, Mercapto, $C_1$-$C_3$ Alkylthio, Carbamoyl oder $C_1$-$C_3$ Alkylcarbamoyl;

$X^1$ Chlor, Brom oder Jod;

$R^1$ Wasserstoff, $C_1$-$C_{18}$ Alkyl oder $C_2$-$C_{18}$ Alkenyl; und W $C_1$-$C_{10}$ Alkylen oder $C_2$-$C_{10}$ Alkenylen darstellen und

m, n und p 0 oder 1 sind, wobei jedoch, wenn m 0 ist, n auch 0 sein muß;

vorausgesetzt daß die Summe der Kohlenstoffatome in den Resten $R^1$ und W größer als 4 ist und 21 nicht überschreitet, daß A und $A^1$ nicht Sulfinyl oder Sulfonyl sein können, wenn X Mercapto bedeutet, und daß, wenn A und $A^1$ Sulfinyl oder Sulfonyl bedeuten, sie auf der gleichen Oxidationsstufe sein müssen;

(D) falls R′ OH ist und R″, R‴ und R″″ H sind, dann

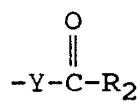

wobei Y ein bivalentes Aminoacylradikal der Formeln

a)

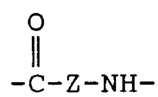

worin Z $C_1$-$C_{10}$ Alkylen oder $C_5$-$C_6$ Cycloalkylen ist,

b)

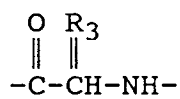

worin $R_3$ Hydroxymethyl, Hydroxyethyl, Mercaptomethyl, Mercaptoethyl, Methylthioethyl, 2-Thienyl, 3-Indolmethyl, Phenyl, Benzyl, Halogenphenyl, Halogenbenzyl, $C_1$-$C_3$ Alkylphenyl, $C_1$-$C_3$ Alkylbenzyl, $C_1$-$C_3$ Alkylthiophenyl, $C_1$-$C_3$ Alkylthiobenzyl, Carbamoylphenyl, Carbamoylbenzyl, $C_1$-$C_3$ Alkylcarbamoylphenyl oder $C_1$-$C_3$ Alkylcarbamoylbenzyl ist; und

c)

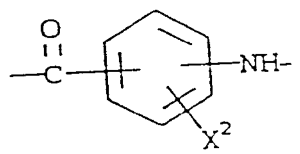

worin $X^2$ Wasserstoff oder Halogen ist; darstellt.

**6.** Verwendung gemäß Anspruch 1, wobei es sich um eine Verbindung mit der Formel

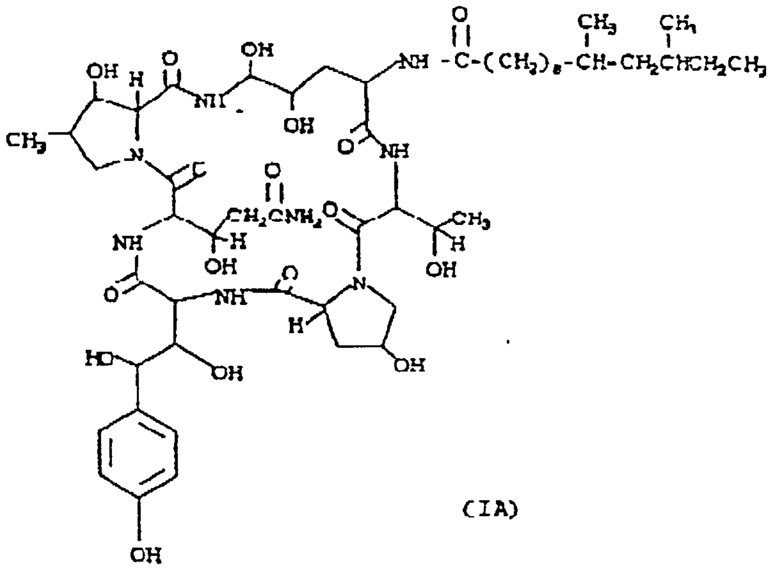

(IA)

handelt.

7. Verwendung gemäß Anspruch 1, wobei die Verbindung 1-[(4R,5R)-4,5-Dihydroxy-$N^2$-(10,12-dimethyl-1-oxotetradecyl)-L-ornithin]-5-(threo-3-hydroxy-1-glutamin)echinocandin B ist.

8. Verwendung gemäß Anspruch 1, wobei die Verbindung 1-[(4R,5R)-4,5-Dihydroxy-$N^2$-[4-(octyloxy)benzoyl]-L-ornithin]-echinocandin B ist.

9. Verwendung gemäß Anspruch 1, wobei die Verbindung Echinocandin B ist.

10. Verwendung gemäß Anspruch 1, wobei die Verbindung Tetrahydroechinocandin B ist.

11. Verwendung gemäß Anspruch 1, wobei die Verbindung O-Methyltetrahydroechinocandin B ist

12. Verwendung gemäß Anspruch 1, wobei die Verbindung O-Benzyltetrahydroechinocandin B ist.

**Patentansprüche für folgende Vertragsstaat : ES**

1. Verfahren für die Herstellung einer Zusammensetzung, die für die Behandlung von Infektionen durch Pneumocystis carinii verwendbar und für die Verabreichung mittels Inhalation angepaßt ist, umfassend die Mischung eines pharmazeutisch annehmbaren Trägers mit einer Cyclohexapeptidverbindung der Formel

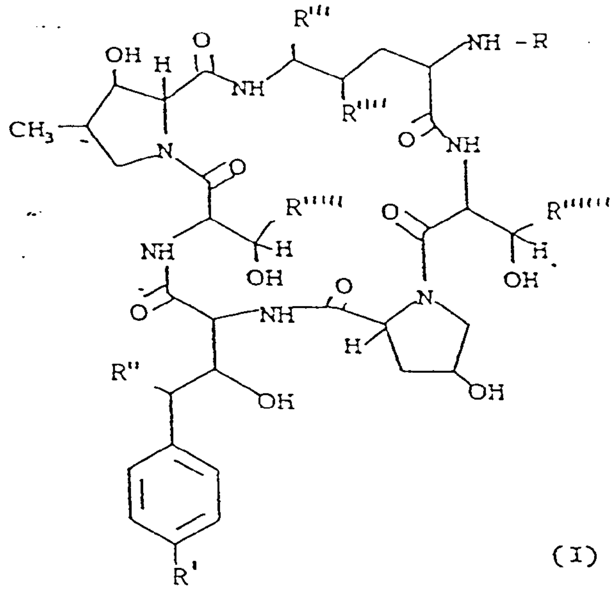

(I)

in der

R′ H oder OH;

R″ H oder OH;

R‴ H, OH, -O-Alkyl($C_1$-$C_6$), -O-Benzyl oder

$$-OCH_2CH_2(CH_2)_mN(R^1)(R^2)\ ;$$

wobei $R^1$ H, $C_1$-$C_{12}$ Alkyl, $C_3$-$C_7$ Cycloalkyl, Hydroxyethyl, $C_{1-6}$ Alkoxyalkyl, Furylmethyl, Tetrahydrofurylmethyl, Phenyl, Phenylalkyl, worin der Phenylrest durch Halogen, Hydroxy, Cl-6 Alkyl oder $C_{1-6}$ Alkoxy substituiert sein kann,

$R^2$ H oder $C_{1-6}$ Alkyl oder

$R^1$ und $R^2$ zusammen den Rest $-(CH_2)_2Q(CH_2)_2-$, in dem Q $-CH_2-$, -NH- oder -N-($C_{1-6}$)Alkyl ist, darstellen und m 0 bis 4 ist;

R⁗ H oder OH;

R‴″ H, $CH_3$ oder $CH_2CONH_2$;

R‴‴ H oder $CH_3$ bedeuten und in der

R eine der folgenden Bedeutungen aufweist:

(A)

$$-C(=O)-Alkyl$$

mit 6 bis 24 Kohlenstoffatomen,

(B)

$$-C(=O)-Alkenyl$$

mit 6 bis 24 Kohlensatoffatomen,

(C) falls R′, R″, R‴ und R″″ OH sind und R‴″ $CH_3$ ist, einen der Reste

(i)

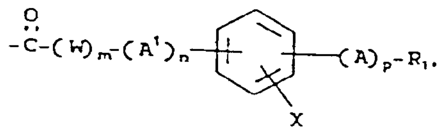

(ii)

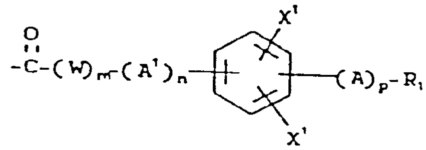

oder

(iii)

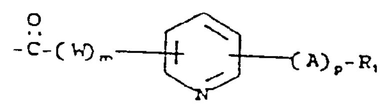

in denen A bivalenten Sauerstoff, Schwefel, Sulfinyl oder Sulfonyl;

$A^1$ bivalenten Sauerstoff, Schwefel, Sulfinyl, Sulfonyl oder -NH-;

X Wasserstoff, Chlor, Brom, Jod, Nitro, $C_1$-$C_3$ Alkyl, Hydroxy, $C_1$-$C_3$ Alkoxy, Mercapto, $C_1$-$C_3$ Alkylthio, Carbamoyl oder $C_{1-3}$ Alkylcarbamoyl;

$X^1$ Chlor, Brom oder Jod;

$R^1$ Wasserstoff, $C_1$-$C_{18}$ Alkyl oder $C_2$-$C_{18}$ Alkenyl;

und W $C_1$-$C_{10}$ Alkylen oder $C_2$-$C_{18}$ Alkenylen darstellen und

m, n und p 0 oder 1 sind, wobei jedoch, wenn m 0 ist, n auch 0 sein muß;

vorausgesetzt daß die Summe der Kohlenstoffatome in den Resten $R^1$ und W größer als 4 ist und 21 nicht überschreitet, daß A und $A^1$ nicht Sulfinyl oder Sulfonyl sein können, wenn X Mercapto bedeutet, und daß, wenn A und $A^1$ Sulfinyl oder Sulfonyl bedeuten, sie auf der gleichen Oxidationsstufe sein müssen;

(D) falls R′ OH ist und R″, R‴ und R″″ H sind, dann

$$-Y-\overset{\displaystyle O}{\overset{\|}{C}}-R_2$$

wobei Y ein bivalentes Aminoacylradikal der Formeln

a)

$$-\overset{\displaystyle O}{\overset{\|}{C}}-Z-NH-$$

worin Z $C_1$-$C_{10}$ Alkylen oder $C_5$-$C_6$ Cycloalkylen ist,

b)

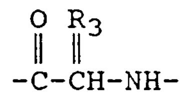

worin $R_3$ Hydroxymethyl, Hydroxyethyl, Mercaptomethyl, Mercaptoethyl, Methylthioethyl, 2-Thienyl, 3-Indolylmethyl, Phenyl, Benzyl, Halogenphenyl, Halogenbenzyl, $C_1$-$C_3$ Alkylphenyl, $C_1$-$C_3$ Alkylbenzyl, $C_1$-$C_3$ Alkylthiophenyl, $C_1$-$C_3$ Alkylthiobenzyl, Carbamoylphenyl, Carbamoylbenzyl, $C_1$-$C_3$ Alkylcarbamoylphenyl oder $C_1$-$C_3$ Alkylcarbamoylbenzyl ist; und

c)

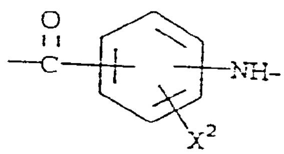

worin $X^2$ Wasserstoff oder Halogen ist; darstellt.

2. Verfahren gemäß Anspruch 1, wobei es sich um eine Verbindung der Formel

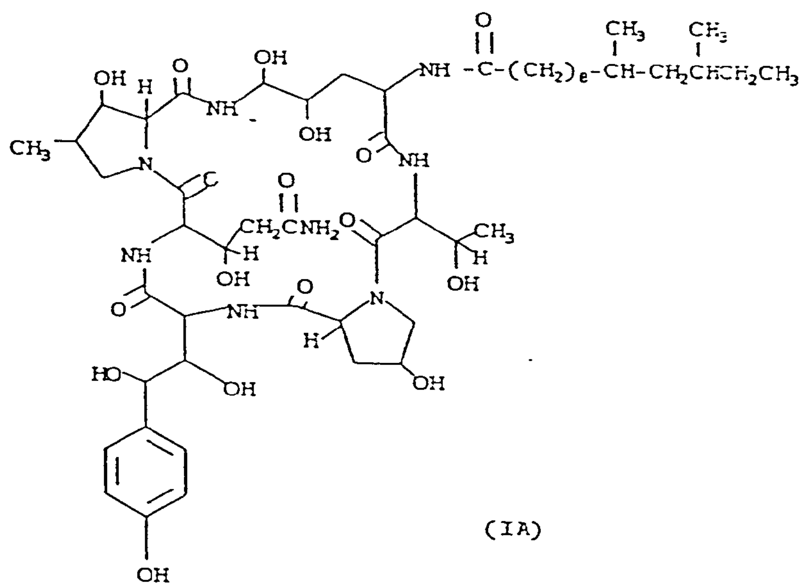

(IA)

handelt.

3. Verfahren gemäß Anspruch 1, wobei die Verbindung 1-[(4R,5R)-4,5-Dihydroxy-$N^2$-(10,12-dimethyl-1-oxotetradecyl)-L-ornithin]-5-(threo-3-hydroxy-1-glutamin)-echinocandin B ist.

4. Verfahren gemäß Anspruch 1, wobei die Verbindung 1-[(4R,5R)-4,5-Dihydroxy-$N^2$-(4-(octyloxy)benzoyl]-L-ornithin]-echinocandin B ist.

5. Verfahren gemäß Anspruch 1, wobei die Verbindung Echinocandin B ist.

6. Verfahren gemäß Anspruch 1, wobei die Verbindung Tetrahydroechinocandin B ist.

7. Verfahren gemäß Anspruch 1, wobei die Verbindung O-Methyltetrahydroechinocandin B ist.

8. Verfahren gemäß Anspruch 1, wobei die Verbindung O-Benzyltetrahydroechinocandin B ist.

## Revendications

## Revendications pour les Etats contractants suivants : AT, BE, CH, DE, FR, GB, IT, LI, LU, NL, SE

1. Utilisation d'un composé cyclohexapeptidique représenté par la formule

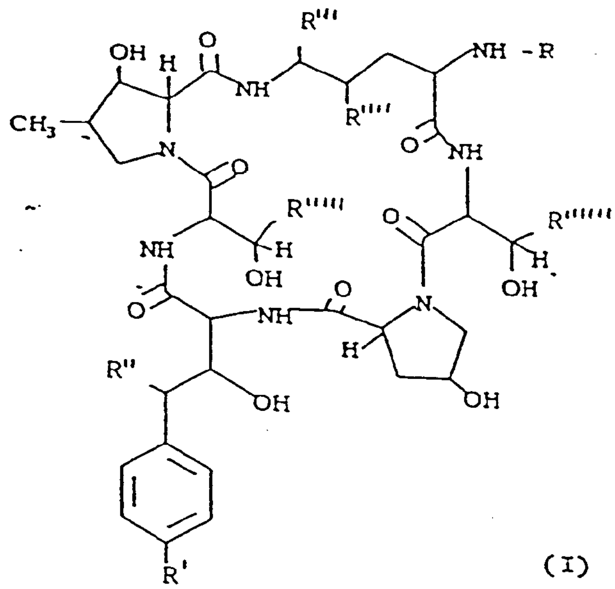

(I)

dans laquelle

R′ est H ou OH ;

R″ est H ou OH ;

R‴ est H, OH, -O-alkyle($C_1$-$C_6$), -O-benzyle ou

$$-OCH_2CH_2(CH_2)_mN\begin{matrix} R^1 \\ R^2 \end{matrix} \quad ;$$

dans laquelle $R^1$ est H, un alkyle en $C_1$-$C_{12}$, un cycloalkyle en $C_3$-$C_7$, un hydroxyéthyle, un (alcoxy en $C_{1-6}$)éthyle, un furylméthyle, un tétrahydrofurylméthyle, un phényle, un phénylalkyle dont le phényle peut être substitué avec un halogéno, un hydroxy, un alkyle en $C_{1-6}$ et un alcoxy en $C_{1-6}$ ; $R^2$ est un hydrogène ou un alkyle en $C_{1-6}$; ou $R^1$ et $R^2$ forment ensemble $-(CH_2)_2Q(CH_2)_2-$ où Q est $-CH_2-$, -NH- ou -N-alkyl($C_{1-6}$)-; et m a une valeur de 0 à 4 ;

R⁗ est H ou OH ;

R‴″ est H, $CH_3$ ou $CH_2CONH_2$ ; et

R‴‴ est H ou $CH_3$

R est

(A)

$$-\overset{\displaystyle O}{\overset{\|}{C}}-\text{alkyle},$$

de 6 à 24 atomes de carbone,

(B)

$$-\overset{\overset{\displaystyle O}{\|}}{C}-\text{alcényle},$$

de 6 à 24 atomes de carbone,

(C), lorsque R′, R″, R‴ et R″″ sont OH et R″‴ est $CH_3$, il est

(i)

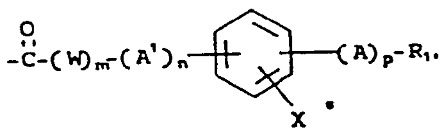

(ii)

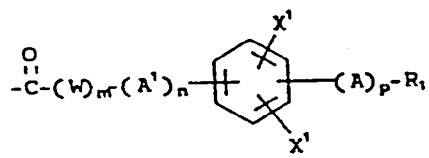

ou

(iii)

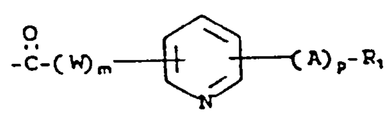

où

A est un oxygène, un soufre, un sulfinyle ou un sulfonyle divalents ;

$A^1$ est un oxygène, un soufre, un sulfinyle ou un sulfonyle divalents ou -NH- ;

X est un hydrogène, un chloro, un bromo, un iodo, un nitro, un alkyle en $C_1$-$C_3$, un hydroxy, un alcoxy en $C_1$- $C_3$ , un mercapto, un alkylthio en $C_1$- $C_3$ , un carbamyle ou un (alkyl en $C_1$- $C_3$)carbamyle ;

$X^1$ est un chloro, un bromo ou un iodo ;

$R^1$ est un hydrogène, un alkyle en $C_1$-$C_{18}$ ou un alcényle en $C_2$-$C_{18}$

W est un alkylène en $C_1$-$C_{10}$ ou un alcénylène en $C_2$-$C_{10}$ ;

m, n et p sont 0 ou 1, mais si m est 0, n doit être 0 ;

sous réserve que la somme des atomes de carbone dans les groupes $R^1$ et W soit supérieure à 4 mais ne puisse pas dépasser 21 ; que, lorsque X est un mercapto, A et $A^1$ ne puissent pas être un sulfinyle ou un sulfonyle ; et que, lorsque A et $A^1$ sont un sulfinyle ou un sulfonyle, ils soient à des états d'oxydation égaux ;

(D), lorsque R′ est OH, R″, R‴ et R″″ sont H, il est

$$-Y-\overset{\overset{\displaystyle O}{\|}}{C}-R_2$$

où Y est un radical aminoacyle divalent de formule

a)

$$-\overset{\overset{\displaystyle O}{\|}}{C}-Z-NH-$$

où Z est un alkylène en $C_1$-$C_{10}$ ou un cycloalkylène en $C_5$-$C_6$

b)

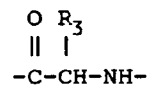

où $R_3$ est un hydroxyméthyle, un hydroxyéthyle, un mercaptométhyle, un mercaptoéthyle, un méthylthioéthyle, un 2-thiényle, un 3-indolométhyle, un phényle, un benzyle, un halogénophényle, un halogénobenzyle, un (alkyl en $C_1$-$C_3$) phényle, un (alkyl en $C_1$-$C_3$)benzyle, un (alkyl en $C_1$-$C_3$) thiophényle, un (alkyl en $C_1$-$C_3$)thiobenzyle, un carbamylphényle, un carbamylbenzyle, un (alkyl en $C_1$-$C_3$) carbamylphényle ou un (alkyl en $C_1$-$C_3$)carbamylbenzyle ;

c)

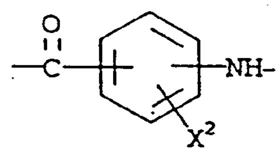

où $X^2$ est un hydrogène ou un halogéno ; pour la préparation d'un médicament utile pour le traitement ou pour la prévention des infections à Pneumocystis carinii.

**2.** Utilisation selon la revendication 1, dans laquelle le composé cyclohexapeptidique est administré à une dose de 0,5 à 20,0 mg/kg de poids corporel de mammifère.

**3.** Utilisation selon la revendication 1, dans laquelle le composé cyclohexapeptidique est administré à un patient dont le système immunitaire est altéré infecté par Pneumocystis carinii.

**4.** Utilisation selon la revendication 1, dans laquelle le composé cyclohexapeptidique est administré à un patient infecté par Pneumocysis carinii et atteint de SIDA.

**5.** Composition utile pour le traitement des infections à Pneumocystis carinii des mammifères et adaptée à l'administration par inhalation, comprenant un véhicule pharmaceutiquement acceptable et un composé cyclohexapeptidique de formule

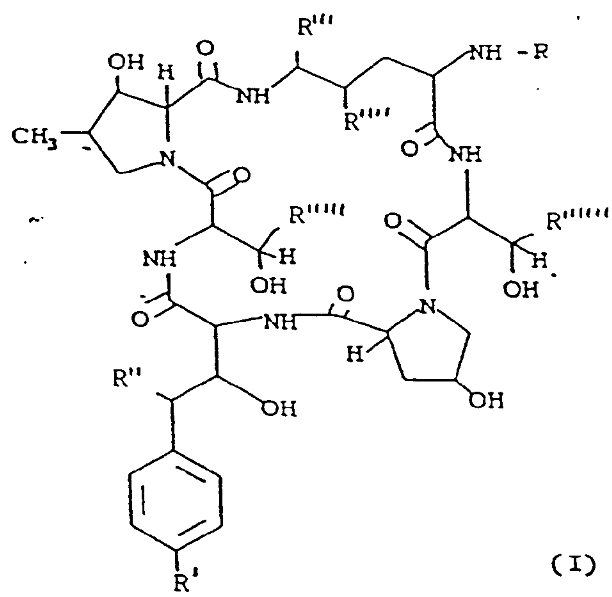

(I)

dans laquelle

R′ est H ou OH ;

R″ est H ou OH ;

R‴ est H, OH, -O-alkyle($C_1$-$C_6$), -O-benzyle ou

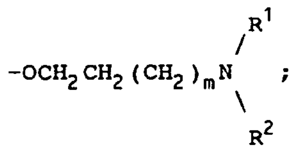

dans laquelle $R^1$ est H, un alkyle en $C_1$-$C_{12}$, un cycloalkyle en $C_3$-$C_7$, un hydroxyéthyle, un (alcoxy en $C_{1-6}$)éthyle, un furylméthyle, un tétrahydrofurylméthyle, un phényle, un phénylalkyle dont le phényle peut être substitué avec un halogéno, un hydroxy, un alkyle en $C_{1-6}$ et un alcoxy en $C_{1-6}$ ; $R^2$ est un hydrogène ou un alkyle en $C_{1-6}$ ; ou $R^1$ et $R^2$ forment ensemble $-(CH_2)_2Q(CH_2)_2-$ où Q est $-CH_2-$, -NH- ou -N-alkyl($C_{1-6}$)- ; et m a une valeur de 0 à 4 ;

R'''' est H ou OH ;

R''''' est H, $CH_3$ ou $CH_2CONH_2$ ; et

R'''''' est H ou $CH_3$

R est

(A)

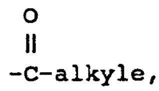

de 6 à 24 atomes de carbone,

(B)

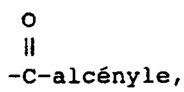

de 6 à 24 atomes de carbone,

(C), lorsque R', R'', R''' et R'''' sont OH et R''''' est $CH_3$, il est

(i)

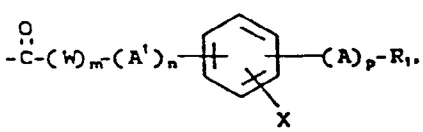

(ii)

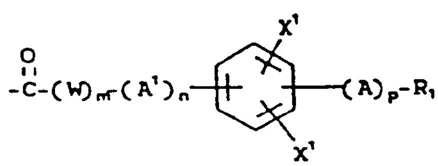

ou

(iii)

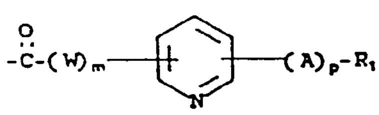

où

A est un oxygène, un soufre, un sulfinyle ou un sulfonyle divalents ;

$A^1$ est un oxygène, un soufre, un sulfinyle ou un sulfonyle divalents ou -NH- ;
X est un hydrogène, un chloro, un bromo, un iodo, un nitro, un alkyle en $C_1$-$C_3$, un hydroxy, un alcoxy en $C_1$-$C_3$ , un mercapto, un alkylthio en $C_1$-$C_3$, un carbamyle ou un (alkyl en $C_1$-$C_3$)carbamyle ;
$X^1$ est un chloro, un bromo ou un iodo ;
$R^1$ est un hydrogène, un alkyle en $C_1$-$C_{18}$ ou un alcényle en $C_2$-$C_{18}$ ;
W est un alkylène en $C_1$-$C_{10}$ ou un alcénylène en $C_2$-$C_{10}$ ;
m, n et p sont 0 ou 1, mais si m est 0, n doit être 0 ;
sous réserve que la somme des atomes de carbone dans les groupes $R^1$ et W soit supérieure à 4 mais ne puisse pas dépasser 21 ; que, lorsque X est un mercapto, A et $A^1$ ne puissent pas être un sulfinyle ou un sulfonyle ; et que, lorsque A et $A^1$ sont un sulfinyle ou un sulfonyle, ils soient à des états d'oxydation égaux ;

(D), lorsque R′ est OH, R″, R‴ et R″″ sont H, il est

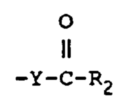

où Y est un radical aminoacyle divalent de formule

a)

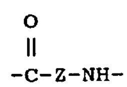

où Z est un alkylène en $C_1$-$C_{10}$ ou un cycloalkylène en $C_5$-$C_6$

b)

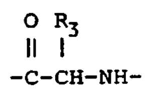

où $R_3$ est un hydroxyméthyle, un hydroxyéthyle, un mercaptométhyle, un mercaptoéthyle, un méthylthioéthyle, un 2-thiényle, un 3-indolométhyle, un phényle, un benzyle, un halogénophényle, un halogénobenzyle, un (alkyl en $C_1$-$C_3$) phényle, un (alkyl en $C_1$-$C_3$)benzyle, un (alkyl en $C_1$-$C_3$) thiophényle, un (alkyl en $C_1$-$C_3$)thiobenzyle, un carbamylphényle, un carbamylbenzyle, un (alkyl en $C_1$-$C_3$) carbamylphényle ou un (alkyl en $C_1$-$C_3$)carbamylbenzyle ;

c)

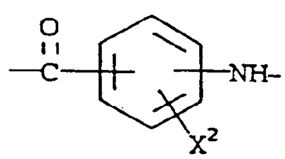

où $X^2$ est un hydrogène ou un halogéno.

6. Utilisation selon la revendication 1, dans laquelle le composé répond à la formule

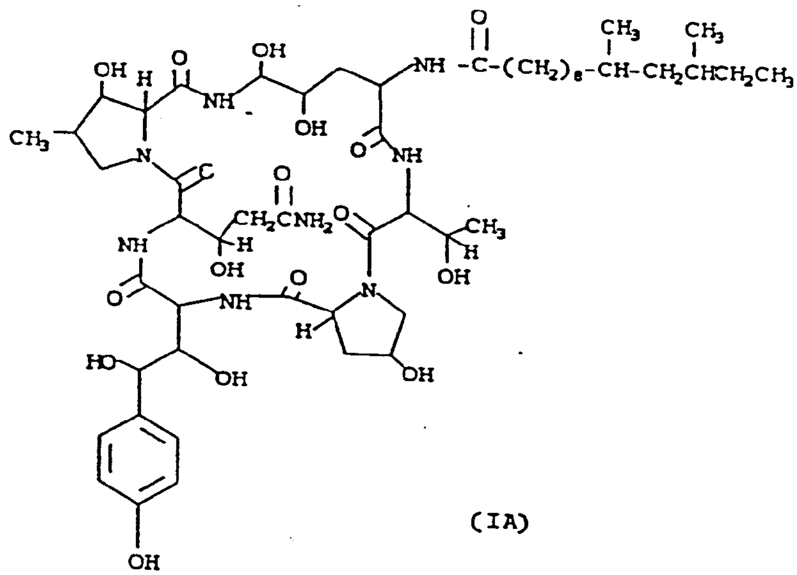

(IA)

7. Utilisation selon la revendication 1, dans laquelle le composé est la 1-[(4R,5R)-4,5-dihydroxy-$N^2$-(10,12-diméthyl-1-oxotétradécyl)-L-ornithine-5-(thréo-3-hydroxy-1-glutamine)] échinocandine B.

8. Utilisation selon la revendication 1, dans laquelle le composé est la 1-[(4R,5R)-4,5-dihydroxy-$N^2$-[4-(octyloxy)benzoyl]-L-ornithine]échinocandine B.

9. Utilisation selon la revendication 1, dans laquelle le composé est l'échinocandine B.

10. Utilisation selon la revendication 1, dans laquelle le composé est la tétrahydroéchinocandine B.

11. Utilisation selon la revendication 1, dans laquelle le composé est l'O-méthyltétrahydroéchinocandine B.

12. Utilisation selon la revendication 1, dans laquelle le composé est l'O-benzyltétrahydroéchinocandine B.

**Revendications pour l'Etat contractant suivant : ES**

1. Procédé pour la préparation d'une composition utile pour le traitement des infections à Pneumocystis carinii des mammifères et adaptée à l'administration par inhalation, qui comprend le mélange d'un véhicule pharmaceutiquement acceptable avec un composé cyclohexapeptidique de formule

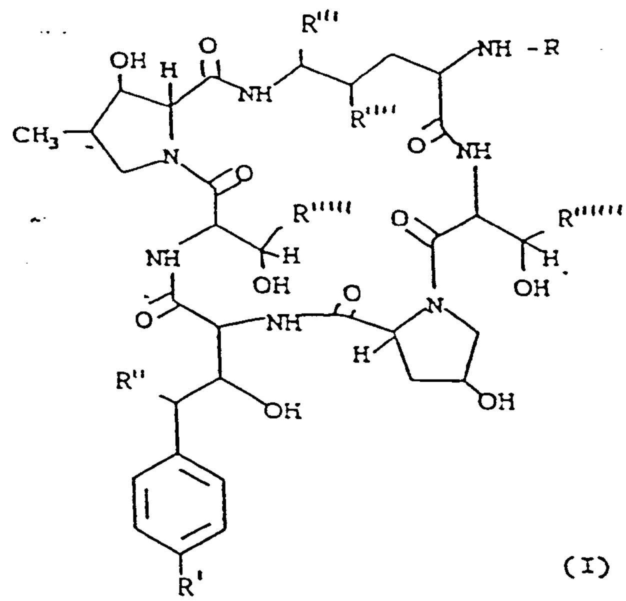

(I)

dans laquelle

R′ est H ou OH ;

R″ est H ou OH ;

R‴ est H, OH, -O-alkyle($C_1$-$C_6$), -O-benzyle ou

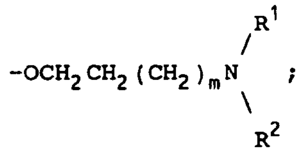

dans laquelle $R^1$ est H, un alkyle en $C_1$-$C_{12}$, un cycloalkyle en $C_3$-$C_7$, un hydroxyéthyle, un (alcoxy en $C_{1-6}$)éthyle, un furylméthyle, un tétrahydrofurylméthyle, un phényle, un phénylalkyle dont le phényle peut être substitué avec un halogéno, un hydroxy, un alkyle en $C_{1-6}$ et un alcoxy en $C_{1-6}$ ; $R^2$ est un hydrogène ou un alkyle en $C_{1-6}$ ; ou $R^1$ et $R^2$ forment ensemble $-(CH_2)_2Q(CH_2)_2-$ où Q est $-CH_2-$, -NH- ou -N-alkyl($C_{1-6}$)- ; et m a une valeur de 0 à 4 ;

R⁗ est H ou OH ;

R‴‴ est H, $CH_3$ ou $CH_2$ $CONH_2$ ; et

R‴‴′ est H ou $CH_3$ ; et

R est

(A)

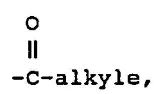

de 6 à 24 atomes de carbone,

(B)

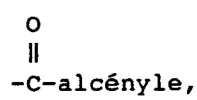

de 6 à 24 atomes de carbone,

(C), lorsque R′, R″, R‴ et R⁗ sont OH et R‴‴′ est $CH_3$, il est

(i)

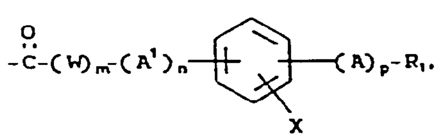

(ii)

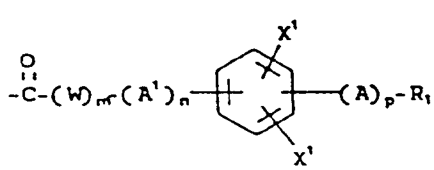

ou

(iii)

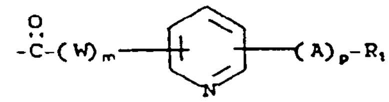

où
A est un oxygène, un soufre, un sulfinyle ou un sulfonyle divalents ;
$A^1$ est un oxygène, un soufre, un sulfinyle ou un sulfonyle divalents ou -NH- ;
X est un hydrogène, un chloro, un bromo, un iodo, un nitro, un alkyle en $C_1$-$C_3$, un hydroxy, un alcoxy en $C_1$-$C_3$ , un mercapto, un alkylthio en $C_1$-$C_3$, un carbamyle ou un (alkyl en $C_1$-$C_3$)carbamyle ;
$X^1$ est un chloro, un bromo ou un iodo ;
$R^1$ est un hydrogène, un alkyle en $C_1$-$C_{18}$ ou un alcényle en $C_2$-$C_{18}$ ;
W est un alkylène en $C_1$-$C_{10}$ ou un alcénylène en $C_2$-$C_{10}$ ;
m, n et p sont 0 ou 1, mais si m est 0, n doit être 0 ;
sous réserve que la somme des atomes de carbone dans les groupes $R^1$ et W soit supérieure à 4 mais ne puisse pas dépasser 21 ; que, lorsque X est un mercapto, A et $A^1$ ne puissent pas être un sulfinyle ou un sulfonyle ; et que, lorsque A et $A^1$ sont un sulfinyle ou un sulfonyle, ils soient à des états d'oxydation égaux ;

(D), lorsque R′ est OH, R″, R‴ et R″″ sont H, il est

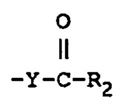

où Y est un radical aminoacyle divalent de formule

a)

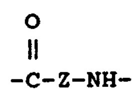

où Z est un alkylène en $C_1$-$C_{10}$ ou un cycloalkylène en $C_5$-$C_6$

b)

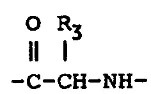

où $R_3$ est un hydroxyméthyle, un hydroxyéthyle, un mercaptométhyle, un mercaptoéthyle, un méthylthioéthyle, un 2-thiényle, un 3-indolométhyle, un phényle, un benzyle, un halogénophényle, un halogénobenzyle, un (alkyl en $C_1$-$C_3$) phényle, un (alkyl en $C_1$-$C_3$) benzyle, un (alkyl en $C_1$-$C_3$) thiophényle, un (alkyl en $C_1$-$C_3$)thiobenzyle, un carbamylphényle, un carbamylbenzyle, un (alkyl en $C_1$-$C_3$) carbamylphényle ou un (alkyl en $C_1$-$C_3$)carbamylbenzyle ;

c)

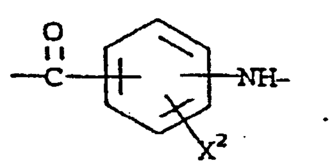

où $X^2$ est un hydrogène ou un halogéno.

**2.** Procédé selon la revendication 1, dans lequel le composé a pour formule

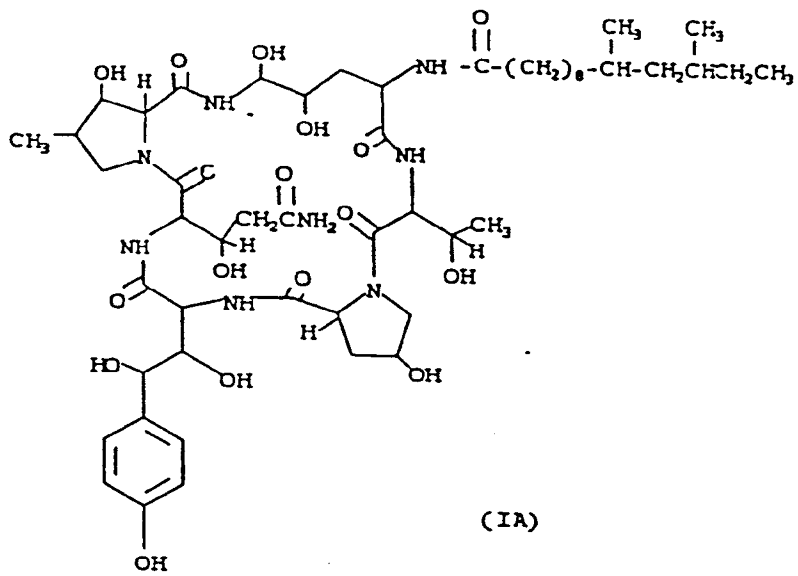

(IA)

3. Procédé selon la revendication 1, dans lequel le composé est la 1-[(4R,5R)-4,5-dihydroxy-N2-(10,12-diméthyl-1-oxo-tétradécyl)-L-ornithine-5-(thréo-3-hydroxy-1-glutamine)]échinocandine B.

4. Procédé selon la revendication 1, dans lequel le composé est la 1-[(4R,5R)-4,5-dihydroxy-N2-[4-(octyloxy)benzoyl]-L-ornithine]échinocandine B.

5. Procédé selon la revendication 1, dans lequel le composé est l'échinocandine B.

6. Procédé selon la revendication 1, dans lequel le composé est la tétrahydroéchinocandine B.

7. Procédé selon la revendication 1, dans lequel le composé est l'O-méthyltétrahydroéchinocandine B.

8. Procédé selon la revendication 1, dans lequel le composé est l'O-benzyltétrahydroéchinocandine B.

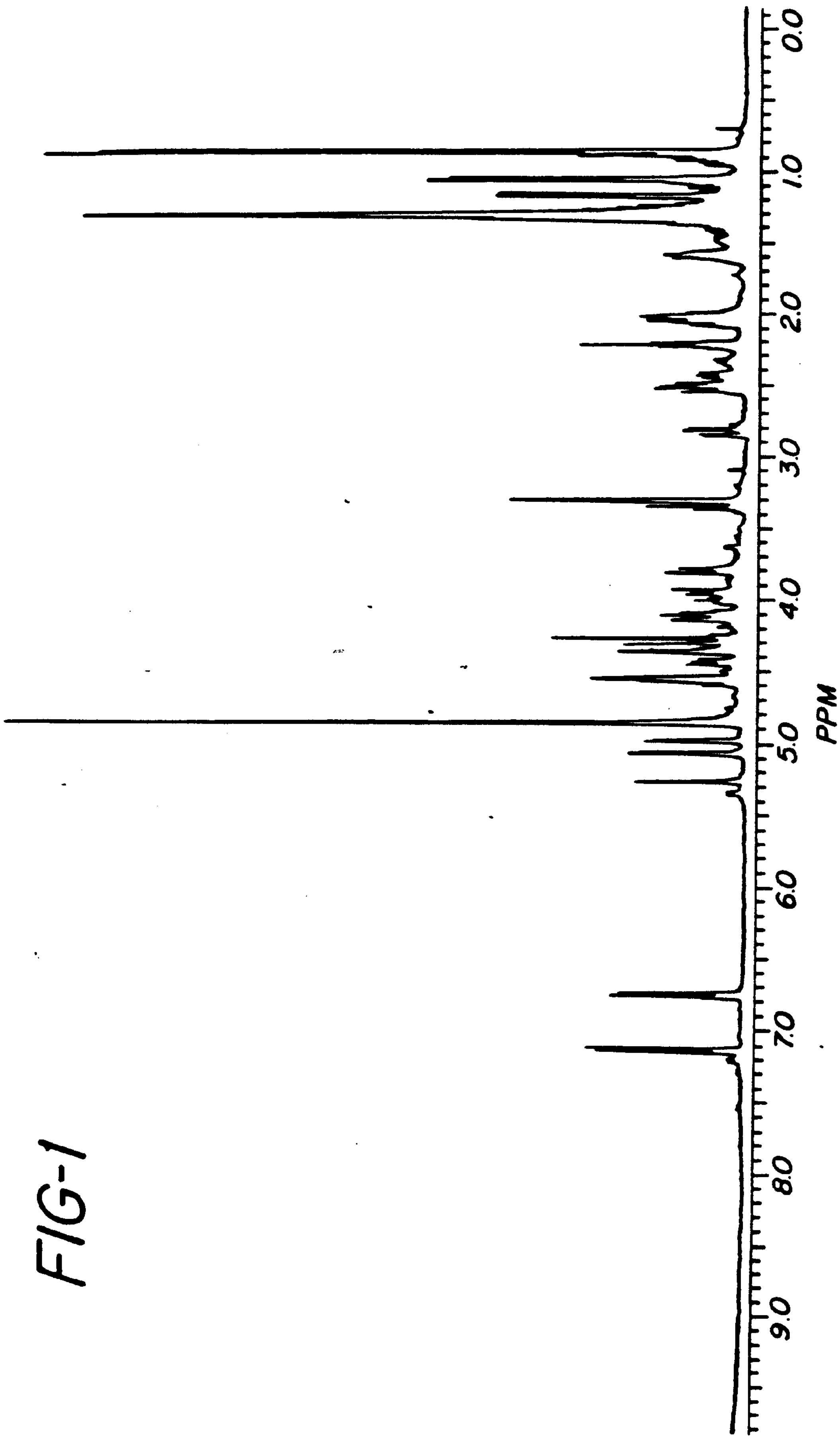


FIG-1

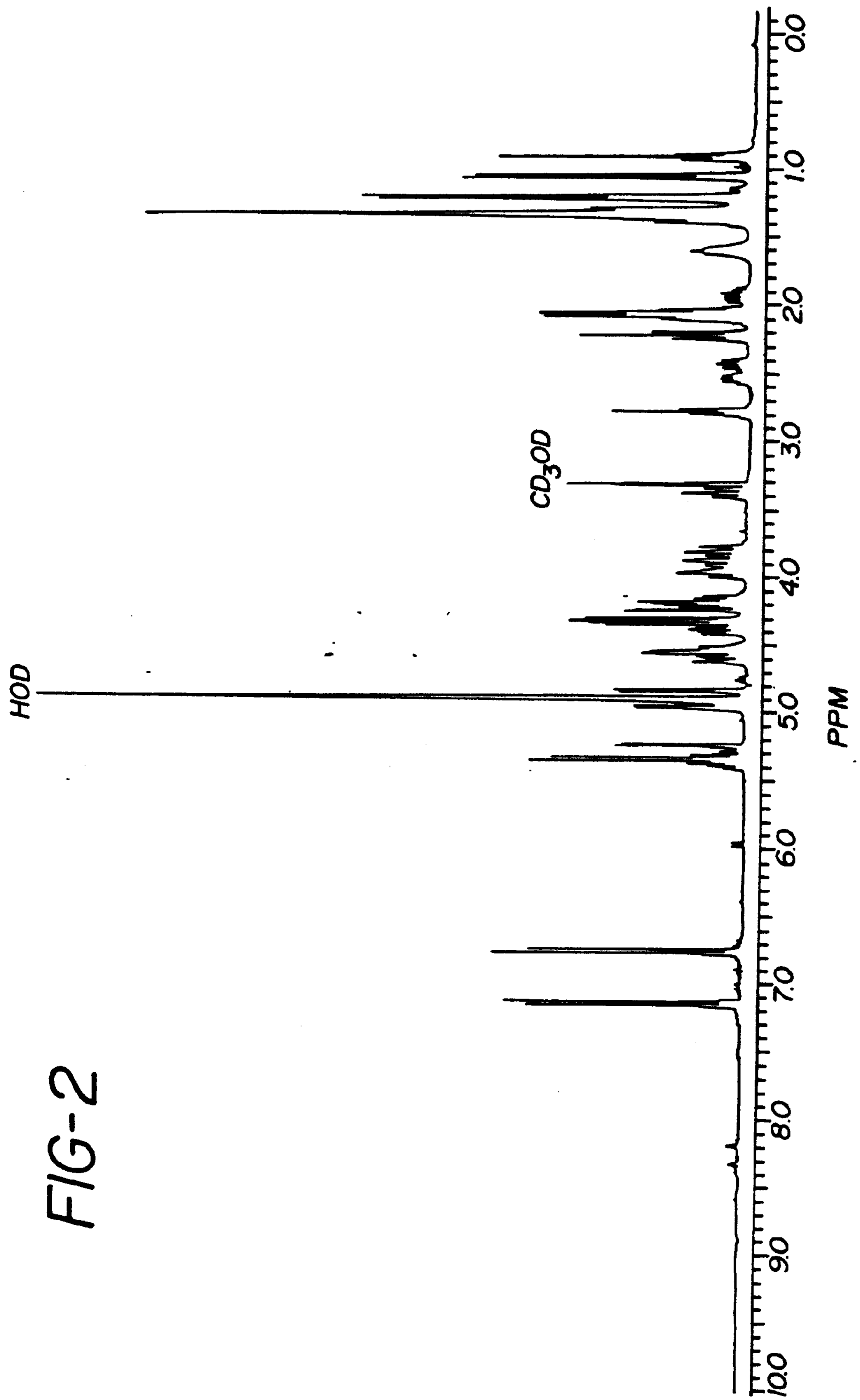


FIG-2